(19) Europäisches Patentamt / European Patent Office / Office européen des brevets

(11) **EP 4 211 458 B1**

(12) **EUROPEAN PATENT SPECIFICATION**

(45) Date of publication and mention
of the grant of the patent:
**29.05.2024  Bulletin 2024/22**

(21) Application number: **21794205.1**

(22) Date of filing: **10.09.2021**

(51) International Patent Classification (IPC):
***G01N 33/00*** (2006.01)

(52) Cooperative Patent Classification (CPC):
**G01N 33/0098;** A01G 7/00

(86) International application number:
**PCT/NL2021/050552**

(87) International publication number:
**WO 2022/055353 (17.03.2022 Gazette 2022/11)**

(54) **METHOD FOR DETERMINING PHYSICAL PARAMETERS OF VASCULAR TISSUE OF A PLANT**

VERFAHREN ZUR BESTIMMUNG PHYSIKALISCHER PARAMETER VON GEFÄSSGEWEBE
EINER PFLANZE

PROCÉDÉ DE DÉTERMINATION DE PARAMÈTRES PHYSIQUES D'UN TISSU VASCULAIRE
D'UNE PLANTE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **11.09.2020  NL 2026453**

(43) Date of publication of application:
**19.07.2023  Bulletin 2023/29**

(73) Proprietors:
• **Technische Universiteit Delft**
  **2628 CN Delft (NL)**
• **Wageningen Universiteit**
  **6708 PB Wageningen (NL)**

(72) Inventors:
• **VERBIEST, Gerard Jan**
  **2600 AA Delft (NL)**
• **STEENEKEN, Peter Gerard**
  **2600 AA Delft (NL)**
• **DUTTA, Satadal**
  **2600 AA Delft (NL)**
• **KAISER, Martin Elias**
  **2600 AA Delft (NL)**
• **MALCOLM MATAMOROS, Priscila Rocío**
  **2600 AA Delft (NL)**

(74) Representative: **EDP Patent Attorneys B.V.**
  **Agro Business Park 2**
  **6708 PW Wageningen (NL)**

(56) References cited:
**EP-A1- 2 359 678     EP-A1- 3 667 312**

• **GUILLAUME CHARRIER ET AL: "Changes in
  ultrasound velocity and attenuation indicate
  freezing of xylem sap", AGRICULTURAL AND
  FOREST METEOROLOGY, ELSEVIER,
  AMSTERDAM, NL, vol. 185, 30 November 2013
  (2013-11-30), pages 20-25, XP028800282, ISSN:
  0168-1923, DOI:
  10.1016/J.AGRFORMET.2013.10.009**
• **BRODERSEN CRAIG R. ET AL: "In vivo
  visualization of the final stages of xylem vessel
  refilling in grapevine ( Vitis vinifera ) stems",
  NEW PHYTOLOGIST, vol. 217, no. 1, 1 January
  2018 (2018-01-01), pages 117-126, XP055807300,
  GB ISSN: 0028-646X, DOI: 10.1111/nph.14811**
• **PAN RUIHUA ET AL: "A comparison of two
  methods for measuring vessel length in woody
  plants", PLANT CELL AND ENVIRONMENT, vol.
  38, no. 12, December 2015 (2015-12), pages
  2519-2526, XP002803075, DOI:
  10.1111/PCE.12566**

EP 4 211 458 B1

**Description**

FIELD OF THE INVENTION

The invention relates to a method for determining a physical vessel parameter of a vascular tissue in a vascular plant. The invention further relates to a system for determining a physical vessel parameter of a vascular tissue in a vascular plant.

BACKGROUND OF THE INVENTION

Methods for characterizing a xylem dimension are known in the art. For instance, Pan et al., "A comparison of two methods for measuring vessel length in woody plants", Plant, Cell and Environment, 2015, describes that vessel lengths are important to plant hydraulic studies, but are not often reported because of the time required to obtain measurements. The article compares a dynamic method with a traditional static method. For both methods, a plant stem segment is cut into pieces.

Guillaume et al., "Changes in ultrasound velocity and attenuation indicate freezing of xylem sap", Agricultural and Forest Meteorology, 2014, describes that (i) the freezing of xylem can be detected by a 1.2-3.6-times increase in ultrasound propagation velocity and decreased wave attenuation, that (ii) attenuation of ultrasounds is reduced depending on water to ice transition and temperature, and that (iii) ultrasounds propagation in wood allows ice detection in tree xylem.

EP2359678A1 describes a method including measuring an occurrence frequency of elastic waves generated by cavitations in vascular tissues in vascular plant, before and after a change in water stress to the vascular plant, respectively by an elastic wave reception sensor fixed to an axis of the vascular plant, calculating a change rate of the occurrence frequency, from the occurrence frequency of the elastic wave measured before and after the change, respectively, and determining whether or not an embolism in the vascular tissue arrives at an unrecoverable level of the embolism, from the calculated change rate.

Brodersen et al., "In vivo visualization of the final stages of xylem vessel refilling in grapevine (Vitis vinifera) stems", New Phytologist, 2017, describes in vivo X-ray micro-computed tomography (microCT) to visualize the final stages of xylem refilling in grapevine (Vitis vinifera) paired with scanning electron microscopy.

EP3667312A1 describes a system including: a vibration exciter that imparts predetermined vibration to a stem or a branch between a fruit and a stalk growing on a plant; a vibration sensor that detects vibration of the stem or the branch caused by the vibration imparted from the vibration exciter; and a detector that detects a weight or weight change of the fruit based on a frequency of the vibration detected by the vibration sensor.

SUMMARY OF THE INVENTION

Water and nutrient transport in plants has attracted much attention in the last decades. In vascular plants, water and nutrient transport is handled by the vascular tissue. In particular, water transport may primarily be handled by xylem vessels, and nutrients produced from photosynthesis may primarily be transported by phloem vessels.

Obtaining a better understanding of plant physiology as a function of age, genetic and environmental parameters is useful for growing and breeding crops, fruits, flowers and trees. In particular, xylem vessels are considered the life-supporting conduits for water and ionic nutrients in plants, and xylem dimensions may be indicative of water carrying capacity and stress resilience. If growers can measure in-plant parameters such as the xylem dimensions, this can be used to optimize growth conditions inside a greenhouse and/or to select suitable plants for further cultivation, both of which may allow a grower to improve yield.

However, prior art techniques for determining a plant's vascular anatomy, such as optical microscopy and electron microscopy, may generally be destructive and time consuming. For example, current techniques may obtain (i) a xylem vessel diameter using optical microscopy, (ii) a xylem vessel length using latex paint-infusion coupled to optical microscopy, (iii) a xylem vessel element length using scanning electron microscopy, and (iv) an elastic modulus of the xylem vessel by uniaxial tensile loading. For all these techniques, the plant may generally need to be cut into pieces to obtain data.

Thereby, with the prior art techniques it may, for example, be particularly challenging, if not practically impossible, to relate the development of a specific plant to the characteristics of its vascular tissue, because, among others, prior art methods may be wasteful as the plants may (at least partially) be damaged or even destroyed, such that their vascular tissue cannot be studied as a function of time.

Hence, it is an aspect of the invention to provide an alternative method for determining a physical vessel parameter of a vascular plant, which preferably further at least partly obviates one or more of above-described drawbacks. The present invention may have as object to overcome or ameliorate at least one of the disadvantages of the prior art, or to provide a useful alternative.

Hence, in a first aspect the invention may provide a method for determining a physical vessel parameter of a vascular tissue in a vascular plant, especially wherein the physical vessel parameter comprises an elasticity or a vessel dimension (of the vascular tissue vessel). The method may comprise one or more of an acoustic excitation stage (also: "excitation stage"), an acoustic detection stage (also "detection stage") and an analysis stage. Hence, in embodiments, the method may comprise an excitation stage. The excitation stage may comprise providing acoustic excitation radiation (also: "excitation radiation") to the vascular plant, especially wherein the acoustic excitation radiation comprises radiation having a frequency selected from the range of 1 - 250 kHz, such as from the range of 10 - 200 kHz. In further embodiments, the method may comprise a detection stage. The detection stage may comprise detecting acoustic emission radiation from the vascular plant, especially acoustic emission radiation from the range of 1 - 250 kHz, such as from the range of 10 - 200 kHz. The detection stage may further comprise providing an emission-related signal. In further embodiments, the method may comprise an analysis stage. The analysis stage may comprise determining the physical vessel parameter based on the emission-related signal. In embodiments, the physical vessel parameter may comprise a viscosity, an elasticity or a vessel dimension, especially an elasticity or a vessel dimension.

Hence, in specific embodiments, the invention may provide a method for determining a physical vessel parameter of a vascular tissue in a vascular plant, wherein the method comprises: an excitation stage comprising providing acoustic excitation radiation to the plant, wherein the acoustic excitation radiation comprises radiation having a frequency selected from the range of 1 - 250 kHz; a detection stage comprising detecting ultrasound emissions from the plant and providing an emission-related signal; and an analysis stage comprising determining the physical vessel parameter based on the emission-related signal, wherein the physical vessel parameter comprises an elasticity or a vessel dimension.

The method of the invention may provide the benefit that a physical vessel parameter of a vascular tissue may be obtained both quickly and non-invasively. Hence, the physical vessel parameter may be obtained without damaging the plant.

In particular, the invention may provide a new platform for determining physical vessel parameters of vascular tissue by implementing a sonographic method. The invention may be based on the observation that remotely recorded ultrasound emissions are a signature of the xylem vessel dimensions and elasticity and represent its resonant modes of vibration. In particular, by recording spontaneous or induced acoustic emission radiation, especially ultrasound emission radiation, of the plant, especially from the vascular tissue, data related to the vessel dimensions and/or vessel elasticities may be obtained in a non-destructive way. The invention may facilitate rapid in-vivo plant phenotyping. The invention may particularly relate to sending an external radiation pulse, especially an ultrasound pulse, through the plant stem and recording induced acoustic emission radiation.

In particular, the invention may provide a non-destructive method for determining one or more of: a (xylem) vessel radius, a (xylem) vessel length, a (xylem) vessel elasticity, and a (xylem) wall thickness, i.e., in embodiments the physical vessel parameter may comprise one or more of a (xylem) vessel radius, a (xylem) vessel length, a (xylem) vessel elasticity, and a (xylem) wall thickness.

The invention will herein, for explanatory purposes, primarily be described in the context of embodiments related to the determination of xylem vessel parameters. It will be clear to the person skilled in the art that the invention is not limited to such embodiments, and that the invention may further apply to the determination of phloem vessel parameters.

Hence, the invention may provide a method for determining a physical vessel parameter of a vascular tissue in a vascular plant (also: "plant").

The term "physical vessel parameter" may herein especially refer to a vessel parameter related to a length, an area, a volume, a thickness, a viscosity and an elasticity. The term "physical vessel parameter" may also refer to a plurality of (different) physical vessel parameters. In embodiments, the physical vessel parameter may be a geometrical parameter.

The term "vascular plant" may herein refer to any plant that has vascular tissue. Further, the term "vascular plant" may herein refer to any plant from the Clade Tracheophytes.

Vascular tissue may be a (specialized) tissue for distributing resources through the vascular plant. Vascular tissue may generally be subdivided into the xylem and phloem, which may be closely associated and may often be arranged directly adj acent to one another in a vascular plant, especially in a vascular bundle. In embodiments, the vascular tissue may especially be the xylem. In further embodiments, the vascular tissue may especially be the phloem.

In embodiments, the method may be a non-destructive method, i.e., the method may not damage, especially not destroy, the vascular plant. In particular, the method may be applied to a whole (live) vascular plant.

The method may especially be an *in vivo* method, i.e., the method may be executed on a whole, living, vascular plant as opposed to only on a tissue extract or a dead vascular plant. The vascular plant may be arranged in a substrate, such as soil. The vascular plant may especially be a whole vascular plant. The vascular plant may especially be a living vascular plant.

In embodiments, the method may comprise one or more of an excitation stage, a detection stage, and an analysis stage.

In further embodiments, the method comprises the excitation stage. The excitation stage may comprise providing acoustic excitation radiation to the vascular plant. In particular, the excitation stage may comprise exposing the vascular plant, especially the vascular tissue, to the acoustic excitation radiation. The term "acoustic excitation radiation" may herein refer to acoustic radiation having a frequency suitable to excite the vascular plant, especially to a vascular tissue of the vascular plant. In particular, the acoustic excitation radiation may comprise a frequency suitable to excite an (internal) resonance of the vascular plant, especially of the vascular tissue, such as of the xylem. It will be clear to the person skilled in the art that the frequency suitable to excite an (internal) resonance of the vascular plant may depend on the type and/or size of the vascular plant. For example, relatively low frequencies may be selected for the acoustic excitation radiation when employing the method in relation to a relatively large plant, such as a tree, whereas relatively large frequencies may be selected when employing the method in relation to a relatively small plant. In particular, in embodiments, the acoustic excitation radiation may especially comprise radiation having a frequency selected from the range of 1 - 250 kHz, such as from the range of 10 - 200 kHz.

In embodiments, the acoustic excitation radiation may comprise radiation having a frequency of at least 1 kHz, such as at least 5 kHz, especially at least 10 kHz, such as at least 20 kHz, especially at least 30 kHz.

In further embodiments, the acoustic excitation radiation may comprise radiation having an (acoustic excitation radiation) frequency of at most 500 kHz, especially at most 250 kHz, such as at most 200 kHz, especially at most 170 kHz, such as at most 150 kHz, especially at most 130 kHz.

In further embodiments, the acoustic excitation radiation frequency and/or acoustic excitation radiation amplitude may be varied in time, especially while the emitted radiation spectra and variations therein are determined to infer properties of the vascular plant, especially of the vascular tissue, or to determine acoustic excitation radiation frequencies at which acoustic emission radiation emission is stimulated most by the acoustic excitation radiation. In further embodiments, excitation pulses (e.g. block pulses) of different duration and amplitudes may be applied, especially where the frequency content depends on the pulse width.

Radiation above 20 kHz may generally be referred to as ultrasound radiation, i.e., radiation with frequencies higher than the upper audible limit of human hearing. In further embodiments, the excitation radiation may be ultrasound excitation radiation, i.e., the excitation radiation may comprise radiation having a frequency in the ultrasound range.

In further embodiments, the method may comprise the detection stage. The detection stage may comprise detecting acoustic emission radiation from the vascular plant and providing an emission-related signal. Especially, the detection stage may comprise detecting resonance acoustic emission radiation from the vascular plant, especially from the vascular tissue, and providing an emission-related signal. It will be clear to the person skilled in the art that, strictly speaking, the measured (or "observed") frequency of acoustic emission radiation may be slightly lower than the true resonant frequency due to non-zero damping.

The term "acoustic emission radiation" may herein refer to acoustic radiation emitted from the vascular plant, especially from the vascular tissue. In particular, the vascular plant, especially the vascular tissue, may have been excited by acoustic excitation radiation in the excitation stage and may subsequently emit acoustic emission radiation. However, the vascular plant, especially the vascular tissue, may also emit acoustic emission radiation without specifically being excited by acoustic excitation radiation. For instance, if a plant experiences drought stress, gas bubbles can nucleate in these vessels, which may result in emission radiation, especially ultrasound emission radiation, being emitted from the plant.

The term "resonance acoustic emission radiation" may herein especially refer to radiation emitted from the vascular plant, especially from the vascular tissue, that matches an (internal) resonance frequency of the vascular tissue. In particular, the acoustic excitation radiation may comprise radiation having a resonance frequency of the vascular tissue, i.e., radiation having a frequency matching a natural frequency of vibration of the vascular tissue, which may cause the vascular tissue to vibrate and emit resonance acoustic emission radiation at the resonance frequency.

The acoustic emission radiation may comprise radiation having the same frequency as the acoustic excitation radiation. The term "frequency" may also refer to a plurality of different frequencies. In particular, in embodiments, the acoustic emission radiation may comprise radiation selected from the same range as the acoustic excitation radiation. Especially, the acoustic excitation radiation and the acoustic emission radiation may overlap in one or more frequencies.

In general, in embodiments, the acoustic emission radiation may be selected from the range of 1 - 250 kHz, such as from the range of 10 - 200 kHz.

In embodiments, the acoustic emission radiation may comprise radiation having a frequency of at least 1 kHz, such as at least 5 kHz, especially at least 10 kHz, such as at least 20 kHz, such as at least 30 kHz.

In further embodiments, the acoustic emission radiation may comprise radiation having a frequency of at most 500 kHz, especially at most 250 kHz, such as at most 200 kHz, especially at most 170 kHz, such as at most 150 kHz, especially at most 130 kHz.

In further embodiments, the emission radiation may be ultrasound emission radiation, i.e., the emission radiation may comprise radiation in the ultrasound range.

The term "emission-related signal" may herein refer to a signal that is related to the detected acoustic emission radiation. In particular, the emission-related signal may comprise raw and/or processed data related to the (detected)

acoustic emission radiation.

In further embodiments, the method may comprise the analysis stage. The analysis stage may comprise determining the physical vessel parameter based on the emission-related signal. In particular, the detected acoustic emission radiation may represent one or more resonant modes of vibration of the dimensions and elasticity of the vascular tissue, such as of a xylem tissue. The resonant modes of vibration may relate to the dimensions and elasticity of the vascular tissue. Hence, the detected acoustic emission radiation may be a signature of the vascular tissue dimensions and elasticity. The physical vessel parameter may especially comprise an elasticity or a vessel dimension, especially an elasticity, or especially a vessel dimension. In particular, the physical vessel parameter may comprise an elasticity or a vessel dimension of the vascular tissue, especially of a vessel (element) of the vascular tissue. In embodiments, the vessel dimension may especially comprise a vessel length or a vessel radius, especially a vessel length, or especially a vessel radius.

In embodiments, the excitation stage and the detection stage may (at least) partially overlap in time. By temporally overlapping the stages, the method may be quicker. In particular, the stages may temporally overlap if the resonance frequencies are of primary interest.

In further embodiments, the excitation stage and the detection stage may be temporally separated, i.e., the excitation stage may be temporally arranged prior to the detection stage. Such embodiments may be particularly relevant when the analysis stage comprises an analysis of time-domain damping because then the acoustic excitation radiation may need to be distinguishable from the detected acoustic emission radiation in the time-domain. In particular, the detection stage may be temporally arranged at a delay after the excitation stage, wherein the delay is equal to or larger than a finite duration of the (pulse of) acoustic excitation radiation.

In embodiments, the vascular tissue may be a xylem tissue, especially a xylem tissue in a plant stem, plant branch or plant root, especially in a plant stem or plant branch. In further embodiments, the vascular tissue may especially be a xylem tissue in an (unearthed) plant root.

The term "plant branch" may herein especially refer to a shoot or stem arising from a (main) axis of the vascular plant, especially a secondary shoot or stem. Hence, the term "plant branch" does not, for example, refer to leaves and/or fruits attached to the plant branch.

In further embodiments, the vascular tissue may be a phloem tissue, especially a phloem tissue in a plant stem, plant branch, or plant root, especially in a plant stem or plant branch.

In particular, the method, especially the excitation stage, may comprise providing acoustic excitation radiation to (or "focusing acoustic excitation radiation on") the plant stem, to the plant branch or to the plant root, especially to the plant stem or the plant branch, more especially to the plant stem, or more especially to the plant branch. In further embodiments, the method, especially the detection stage, may comprise (specifically) detecting ultrasound emission radiations from the plant stem or the plant branch, especially from the plant stem, or especially from the plant branch.

In embodiments, the vascular plant, especially the plant stem, or especially the plant branch, may have a stem diameter selected from the range of 0.5 - 20 mm, such as from the range of 1 - 10 mm.

In embodiments, the vascular plant may be a monocot plant or a dicot plant, especially a monocot plant, or especially a dicot plant. The term "monocot plant" may especially refer to a member of the monocotyledons. The term "dicot plant" may especially refer to a member of the dicotyledons. In further embodiments, the vascular plant may be a herbaceous plant or a woody plant. The term "herbaceous plant" may especially refer to a plant having a non-woody stem, whereas the term "woody plant" may especially be a plant having a woody stem. In further embodiments, the vascular plant may especially be a herbaceous dicot plant.

As indicated above, the vascular plant may also (spontaneously) emit some acoustic emission radiation in the absence of dedicated acoustic excitation radiation, such as when the vascular plant experiences drought. Hence, in specific embodiments, the invention may provide a method for determining a physical vessel parameter of a vascular tissue in a vascular plant, wherein the method comprises: a detection stage comprising detecting ultrasound emissions from the plant and providing an emission-related signal; and an analysis stage comprising determining the physical vessel parameter based on the emission-related signal, wherein the physical vessel parameter comprises an elasticity or a vessel dimension.

In particular, in further specific embodiments, the acoustic emission radiation may comprise radiation selected from the range of 10 - 100 kHz, wherein the vascular tissue is a xylem tissue in a plant stem or plant branch, and wherein the vascular plant is a herbaceous dicot plant having a stem diameter (of the plant stem or the plant branch) selected from the range of 1 - 10 mm.

The vascular tissue, especially the xylem, may have a longitudinal axis. In particular, in general, the longitudinal axis may be parallel to an axis of elongation of a plant stem and/or a plant branch (comprising the vascular tissue).

In embodiments, the detection stage may comprise detecting acoustic emission radiation from the vascular plant at a first location arranged axially with respect to the longitudinal axis, i.e., the first location may essentially be arranged on the longitudinal axis. Such an arrangement may facilitate identifying radiation, especially a sound wave, that resonates and propagates along the length of the vessels. The frequencies may be governed by the vessel length, while the damping (in the sound) in time-domain may relate to the vessel radii and/or the kinematic viscosity of the sap present in the vessels.

In particular, especially with regards to xylem, the acoustic emission radiation measured from the first location may be informative with regards to one or more of a xylem vessel (element) length L, a xylem radius R, an elastic modulus E, a sap (water) density $\rho_l$, a bulk compressibility of sap K, a xylem wall thickness h, and a dynamic viscosity $\eta_l$. In particular, the acoustic emission radiation detected at the first location may comprise an observed $m^{th}$ order (resonance) frequency $f_m$, wherein $f_m$ may (approximately) equal $m/2*v_{eff}/L$, i.e.,:

$$f_m \approx \left(\frac{m}{2}\right) * \frac{v_{eff}}{L}$$

wherein m is the mode order, and wherein:

$$v_{eff} \approx \sqrt{\frac{(\frac{1}{K} * \frac{2*R}{h*E})^{-1}}{\rho_l}}$$

Further, the acoustic emission radiation detected at the first location may dampen (in the time domain), wherein the dampening may be characterized by an emission radiation settling time $\tau_s$, wherein:

$$R \approx \sqrt{\frac{4 * \eta_l * \tau_s}{\rho_l}}$$

Hence, if, at the first location, the frequency of the observed acoustic emission radiation from a vascular tissue is higher than that of a reference vascular tissue, this may be indicative of the vascular tissue having a higher value for one or more of an elastic modulus E, a bulk compressibility of sap K, a xylem wall thickness h, or a lower value for one or more of a xylem vessel (element) length L, a xylem radius R or a sap (water) density $\rho_l$. Similarly, if the settling time $\tau_s$ of the observed acoustic emission radiation from a vascular tissue is higher than that of a reference vascular tissue, this may be indicative of the vascular tissue having a higher value for one or more of the xylem radius and the sap (water) density $\rho_l$, or of a lower value of the dynamic viscosity $\eta_l$.

In further embodiments, the detection stage may comprise detecting acoustic emission radiation from the vascular plant at a second location arranged perpendicular (or "arranged radially") with respect to the longitudinal axis, i.e., the second location may be arranged along the plant stem (or the plant branch) and perpendicular to the longitudinal axis. Such an arrangement may facilitate identifying radiation, especially a sound wave, that is generated by the flexural and/or radial vibration modes of a vessel-sap composite. The corresponding frequencies may be governed by one or more of the length, radius, and elastic modulus of the vessels.

In particular, especially with regards to xylem, the acoustic emission radiation measured from the second location may be informative with regards to one or more of a xylem vessel (element) length L, a xylem radius R, an elastic modulus E, a xylem mass density $\rho_{xylem}$, and a solid viscoelasticity $\eta_{solid}$. In particular, the acoustic emission radiation detected at the second location may comprise an observed $n^{th}$ order (resonance) frequency $f_n$, wherein:

$$f_n \approx \left(\frac{k_T^2}{4\pi}\right) * \left(\frac{R}{L^2}\right) * \sqrt{\frac{E}{\rho_{xylem}}}$$

wherein $k_T$ is a mode constant, i.e., a pre-factor that occurs in the expression of the resonance frequency of a vibrating beam. The value may depend of the order "n". In particular, in embodiments, for n = 1, 2, or 3, $k_T$ may be 4.73, 7.8532, or 10.996 respectively. For n >3, $k_T$ may be about $(n+0.5)*\pi$. The correct mode constant may be known by identifying which set of theoretical characteristic frequencies is a close match with the observed set of characteristic frequencies. Further, the acoustic emission radiation detected at the first location may dampen (in the time domain), wherein the dampening may be characterized by a settling time $\tau_s$, wherein:

$$\tau_s \approx \frac{\eta_{solid}}{E}$$

Hence, if, at the second location, the frequency of the observed acoustic emission radiation from a vascular tissue is higher than that of a reference vascular tissue, this may be indicative of the vascular tissue having a higher value for one or more of the elastic modulus E and a xylem radius R, or a lower value for one or more of a xylem vessel (element) length L, and a xylem mass density $\rho_{xylem}$. Similarly, if the settling time $\tau_s$ of the observed acoustic emission radiation from a vascular tissue is higher than that of a reference vascular tissue, this may be indicative of the vascular tissue having a higher value for the solid viscoelasticity $\eta_{solid}$, or a lower value for the elastic modulus E.

It will be clear to the person skilled in the art that the physical vessel parameters may further vary as a function of other (measurable) features. For example, the elastic modulus and the xylem mass density may also depend on water/moisture content of the xylem tissue.

In particular, in further embodiments, the detection stage may comprise detecting acoustic emission radiation from the vascular plant at both the first location and the second location.

In embodiments, the excitation stage may comprise providing acoustic excitation radiation via a pulse, especially wherein the pulse has a pulse duration that is larger than a characteristic settling time due to damping in the vascular tissue. In particular, the pulse duration may be larger than 1 ms. In further embodiments, the pulse duration may be selected from the range of 0.5 - 10 ms, such as from the range of 1-10 ms. In further embodiments, the pulse duration may be at least 0.5 ms, such as at least 1 ms, especially at least 1.1 ms, such as at least 1.2 ms, especially at least 1.5 ms, such as at least 2 ms. In further embodiments, the pulse duration may be at most 20 ms, such as at most 15 ms, especially at least 10 ms, such as at most 5 ms, especially at most 3 ms, such as at most 2 ms. In further embodiments, the excitation stage may comprise providing the pulse via a step excitation or a narrow rectangular pulse. The settling time may especially be determined by fitting an amplitude-envelope to a time-domain signal with a single exponential function.

In further embodiments, the pulse may have a pulse duration smaller than the time-of flight (propagation time) of the acoustic excitation radiation through the plant, such as through the plant stem, especially through the vascular tissue. Such an embodiment may be beneficial as it may prevent a temporal overlap between acoustic excitation radiation and acoustic emission radiation exiting the plant. In particular, such embodiments may be beneficial when the damping (over time) of the acoustic emission radiation is used to determine the physical vessel parameter

In particular, the excitation stage may comprise providing acoustic excitation radiation via a pulse having a pulse duration $T_{ON}$, and only a single pulse may be provided during a delay time $T_{delay}$. The acoustic excitation radiation may have an acoustic excitation radiation settling time $\tau$, which may be determined by fitting an amplitude-envelope to an (observed) time-domain signal with a single exponential function. Similarly, the acoustic emission radiation may have an acoustic emission radiation settling time $\tau_s$, which may be determined by fitting an amplitude-envelope to an (observed) time-domain signal with a single exponential function. In embodiments, $T_{delay} > \tau_s$, which may provide the benefit that the excitation of the vascular tissue due to a first pulse may be (mostly) settled down before the next pulse excites the vascular tissue. In further embodiments, $\tau > \tau_s$, which may serve to prevent the settling time in the plant response to be limited by the settling time of the acoustic excitation radiation. In further embodiments, $T_{ON} < 1/f_{plant}$, wherein $f_{plant}$ is the highest resonance frequency of interest, such that a bandwidth of the acoustic excitation radiation extends above the highest resonance frequency of interest $f_{plant}$.

In embodiments, the analysis stage may comprise fitting at least part of the emission-related signal to a model of flexural modes (or: "bending modes") of a cylindrical beam (also see below), and determining the physical vessel parameter (at least partially) based on the model. The cylindrical beam may especially represent the vascular tissue.

In further embodiments, the analysis stage may comprise fitting an exponential decay curve to (the amplitude of) at least part of the emission-related signal (in the time domain). The analysis stage may further comprise determining a decay parameter based on the exponential decay curve, especially wherein the physical vessel parameter is determined based on the decay parameter. In particular, the decay parameter may be fed to (or "provided to" or "incorporated in") the model, wherein the physical vessel parameter is determined based on the model, especially based on the model (parameterized) with the decay parameter. In such embodiments the physical vessel parameter may especially comprise one or more of a vascular tissue radius, a sap viscosity and a vascular tissue (visco)elasticity, more especially one or more of a xylem vessel radius, a sap viscosity, and a xylem (visco)elasticity.

In embodiments, the detected acoustic emission radiation may be converted into the frequency domain, such as via a Fourier transformation. In particular, the analysis stage may comprise converting data in the emission-related signal to the frequency domain.

In further embodiments, the analysis stage may comprise determining one or more peaks in at least part of the emission-related signal in the frequency domain, especially wherein the physical vessel parameter is determined based on the one or more peaks. In particular, the (frequencies and/or amplitudes corresponding to the) one or more peaks may be fed to (or "provided to" or "incorporated in") the model, wherein the physical vessel parameter is determined based on the model, especially based on the model (parameterized) based on the one or more peaks. In such embodiments, the physical vessel parameter may especially comprise one or more of a vascular tissue vessel element length, and a Young's modulus (of the vascular tissue), especially a xylem vessel element length, and a Young's modulus (of the xylem).

The term "xylem vessel element" may refer to a tubular compartment (or "element" comprised by the xylem vessel, i.e., the xylem vessel may be composed of a series-network of tubular compartments. The xylem vessel elements of a xylem vessel may be separated by perforation plates. Hence, a xylem vessel element may be defined by (the presence of) perforation plates at each end. The xylem vessel elements may define the resonating unit for the ultrasound. Hence, in embodiments, the physical vessel parameter may be a physical vessel parameter of a xylem vessel element. The perforation plates may be modeled as non-ideal (lossy) reflecting boundaries.

The physical vessel parameter may be indicative of plant performance, which may further relate to plant well-being. In particular, the physical vessel parameter may be indicative of the quality of one or more of plant growth conditions and/or plant genetics, and especially also the interplay between plant growth conditions and plant genetics (Genetics x Environment Interaction). For example, the physical vessel parameter may suggest that the vascular plant has an excess or lack of, for example, water, a nutrient, or lighting.

Hence, in embodiments, the method may further comprise controlling a plant cultivation condition based on the physical vessel parameter, especially wherein the plant cultivation condition is selected from the group comprising a watering regime, a lighting regime, and a nutrient regime.

In further embodiments, the method may comprise determining (values of) the physical vessel parameter for a plurality of vascular plants, wherein the method further comprises selecting one or more vascular plants of the plurality of vascular plants for breeding based on (the values of) the physical vessel parameter. For instance, the method may be employed for breeding drought-resistant vascular plants by exposing a plurality of vascular plants to drought, and: determining the physical vessel parameter of the plurality of vascular plants at one or more points in time; determining a performance parameter of the vascular plants based on the physical vessel parameter; and selecting one or more vascular plants of the plurality of vascular plants for breeding based on the performance parameter (or directly based on the physical vessel parameter).

The vascular plant, especially the vascular tissue, may have a plurality of resonance frequencies. Hence, the method, especially the excitation stage, may comprise providing acoustic excitation radiation comprising radiation having a plurality of frequencies, especially a plurality of frequencies selected from the range of 1 - 250 kHz. Similarly, the method, especially the detection stage, may comprise detecting acoustic emission radiation comprising radiation having a plurality of frequencies, especially a plurality of frequencies selected from the range of 1 - 250 kHz.

In embodiments, the method, especially the excitation stage, may comprise providing acoustic excitation radiation comprising broadband radiation having one or more frequencies in the range of 1 - 250 kHz, such as in the range of 10 - 200 kHz, especially in the range of 10 - 150 kHz. In particular, the method may comprise providing broadband acoustic excitation radiation comprising one or more frequencies in the range of 1 - 250 kHz, especially in the range of 10 - 150 kHz

In embodiments, the method may comprise varying the frequency of the radiation in the acoustic excitation radiation and/or in the acoustic emission radiation. In particular, the method may comprise executing a frequency sweep. The term "frequency sweep" may herein especially refer to (temporally) varying an excitation or emission frequency. A frequency sweep may be insightful as to the response of the vascular plant with regards to specific frequencies.

Hence, in further embodiments, the excitation stage may comprise an excitation frequency sweep from a first frequency to a second frequency, i.e., wherein the frequency of the (radiation of the) acoustic excitation radiation is varied during one or more excitation stages. In particular, the method may comprise a plurality of excitation stages, wherein the acoustic excitation radiation of different excitation stages comprises different frequencies.

In particular, the method may further comprise one or more detection stages, especially wherein each excitation stage is followed by a respective detection stage. Hence, the method may comprise a plurality of sets of stages, wherein each set comprises an excitation stage and a detection stage. In further embodiments, each set may further comprise an analysis stage. However, the method may also comprise a single (continuous) analysis stage to analyze the emission-related data of the plurality of detection stages.

Hence, in further embodiments, the method may comprise a plurality of (sets comprising respective) excitation stages and detection stages, and a (single) analysis stage.

In embodiments, only a single pulse may be provided during a delay time $T_{delay}$, i.e., two consecutive pulses, especially provided in consecutive excitation stages, may be temporally separated by a delay time $T_{delay}$. In further embodiments, $T_{delay}$ may be larger than an acoustic emission radiation settling time In further embodiments, the delay time $T_{delay}$ may be selected from the range of 0.5 - 10 ms, such as from the range of 1-10 ms. In further embodiments, $T_{delay}$ may be at least 0.5 ms, such as at least 1 ms, especially at least 1.1 ms, such as at least 1.2 ms, especially at least 1.5 ms, such as at least 2 ms. Similarly, in further embodiments, the detection stage may comprise an emission frequency sweep from the first frequency to the second frequency, i.e., the detection stage may comprise sweeping an emitted pulse profile. In particular, the emission frequency sweep may comprise varying a detection frequency during one or more detection stages.

In further embodiments, the first frequency and the second frequency may be selected from the range of 1-250 kHz, such as from the range of 10-150 kHz. The first frequency and the second frequency may especially be at least 5 kHz apart, such as at least 10 kHz apart, especially at least 20 kHz apart, such as at least 50 kHz apart.

In a second aspect, the invention may further provide a system for determining a physical vessel parameter of a vascular tissue in a vascular plant. The system may comprise an acoustic radiation device (also "radiation device"), especially an ultrasound device, and a control system. In embodiments, the acoustic radiation device may comprise a radiation generation device, especially an ultrasound generation device. In further embodiments, the acoustic radiation device may comprise a radiation detection device, especially an ultrasound detection device. In specific embodiments, the radiation generation device and the radiation detection device may be the same device. In embodiments, the system may have an operational mode. The operational mode may comprise one or more of an excitation stage, a detection stage and an analysis stage. In embodiments, the operational mode may comprise the excitation stage. In the excitation stage, the acoustic radiation device may be configured to provide acoustic excitation radiation (to the vascular plant), especially wherein the acoustic excitation radiation comprises radiation having a frequency selected from the range of 1-250 kHz, such as from the range of 10 - 150 kHz. In further embodiments, the operational mode may comprise the detection stage. In the detection stage, the acoustic radiation device may be configured to detect (resonance) acoustic emission radiation (from the vascular plant) and to provide an emission-related signal to the control system. In further embodiments, the operational mode may comprise an analysis stage. In the analysis stage, the control system may be configured to determine the physical vessel parameter based on the emission-related signal, especially wherein the physical vessel parameter comprises an elasticity or a vessel dimension.

In embodiments, the radiation generation device may especially be configured to provide acoustic excitation radiation comprising radiation having a frequency selected from the range of 1 - 250 kHz, such as from the range of 10 - 200 kHz. In further embodiments, the radiation generation device may be configured to provide acoustic excitation radiation comprising radiation having a frequency of at least 1 kHz, such as at least 5 kHz, especially at least 10 kHz, such as at least 20 kHz, such as at least 30 kHz. In further embodiments, the radiation generation device may be configured to provide acoustic excitation radiation comprising radiation having a frequency of at most 500 kHz, especially at most 250 kHz, such as at most 200 kHz, especially at most 170 kHz, such as at most 150 kHz, especially at most 130 kHz.

In specific embodiments, the acoustic radiation device may comprise a radiation detection device. In further embodiments, the acoustic radiation device may be a radiation detection device.

In embodiments, the radiation detection device may especially be configured to detect acoustic emission radiation comprising radiation having a frequency selected from the range of 1 - 250 kHz, such as from the range of 10 - 200 kHz. In further embodiments, the radiation detection device may be configured to detect acoustic emission radiation comprising radiation having a frequency of at least 1 kHz, such as at least 5 kHz, especially at least 10 kHz, such as at least 20 kHz, such as at least 30 kHz. In further embodiments, the radiation detection device may be configured to detect acoustic emission radiation comprising radiation having a frequency of at most 500 kHz, especially at most 250 kHz, such as at most 200 kHz, especially at most 170 kHz, such as at most 150 kHz, especially at most 130 kHz.

In specific embodiments, the invention may provide a system for determining a physical vessel parameter of a vascular tissue in a vascular plant, wherein the system comprises an acoustic radiation device and a control system, wherein the system has an operational mode, wherein the operational mode comprises: an excitation stage comprising the acoustic radiation device providing acoustic excitation radiation to the vascular plant, wherein the acoustic excitation radiation comprises radiation having a frequency selected from the range of 1-250 kHz; a detection stage comprising the acoustic radiation device detecting acoustic emission radiation from the vascular plant and providing an emission-related signal to the control system; and an analysis stage comprising the control system determining the physical vessel parameter based on the emission-related signal, wherein the physical vessel parameter comprises an elasticity or a vessel dimension.

In embodiments, the radiation generation device and the radiation detection device may be a single device.

The system may especially be configured to execute the method of the invention.

Hence, in specific embodiments, the invention may provide a system for determining a physical vessel parameter of a vascular tissue in a vascular plant, wherein the system comprises an acoustic radiation device and a control system, wherein the system has an operational mode, wherein the operational mode comprises: a detection stage comprising the acoustic radiation device detecting acoustic emission radiation from the vascular plant and providing an emission-related signal to the control system; and an analysis stage comprising the control system determining the physical vessel parameter based on the emission-related signal, wherein the physical vessel parameter comprises an elasticity or a vessel dimension. In further embodiments, the acoustic emission radiation may comprise radiation selected from the range of 10 - 100 kHz, the vascular tissue may be a xylem tissue in a plant stem, plant branch or plant root (if isolated from soil), especially a xylem tissue in a plant stem or plant branch, and the vascular plant may be a herbaceous dicot plant having a stem diameter (of the plant stem or the plant branch) selected from the range of 1 - 10 mm.

Hence, in embodiments, the acoustic radiation device, especially the radiation detection device, may be configured to detect acoustic emission radiation in the range of 1 - 250 kHz, such as in the range of 10 - 200 kHz.

In further embodiments, the acoustic radiation device, especially the radiation detection device, may have a characteristic settling time that is larger than the settling time (due to damping) of the vascular tissue, especially at least 5 times larger than the settling time, such as at least 10 times larger than the settling time.

In embodiments, the system may comprise a stem mount configured for attaching the acoustic radiation device to a plant stem or a plant branch of the vascular plant, especially to a plant stem, or especially to a plant branch. In such embodiments, the operational mode may comprise providing excitation radiation to the plant stem or the plant branch and detecting acoustic emission radiation from the plant stem or the plant branch, especially to and from the plant stem, or especially to and from the plant branch.

In further embodiments, the acoustic radiation device may comprise an axial radiation detection device and/or a radial radiation detection device, especially an axial radiation detection device, or especially a radial radiation detection device. In such embodiments, in the operational mode: the axial radiation detection device may be arranged at a first location arranged axially along the longitudinal axis; and/or the axial radiation detection device may be arranged at a second location arranged perpendicular to the longitudinal axis.

In further embodiments, the system may further comprise a measurement site configured for hosting the vascular plant, wherein the acoustic radiation device comprises an axial radiation detection device arranged at a first location (in the measurement site) arranged axially along the longitudinal axis; and/or wherein the acoustic radiation device comprises an axial radiation detection device arranged at a second location (in the measurement site) arranged perpendicular to the longitudinal axis.

In further embodiments, the system may be configured to move the acoustic radiation device relative to the vascular plant, especially to perform measurements at multiple locations. Hence, in embodiments, in the detection stage, the system may move the acoustic radiation device to a plurality of positions relative to the vascular plant, such as to the first location and to the second location. Hence, in embodiments, the acoustic radiation device, especially the radiation detection device, may be arranged on a robot arm.

In embodiments, the excitation stage may comprise the acoustic radiation device providing the acoustic excitation radiation via a pulse. In further embodiments, the pulse may have a pulse duration that is larger than a characteristic settling time of the vascular tissue. In further embodiments, the pulse may have a pulse duration smaller than a time-of-flight of the acoustic excitation radiation through the vascular tissue.

In embodiments, the acoustic radiation device, especially the radiation generation device, may be configured to provide broadband radiation. Hence, in embodiments, the acoustic excitation radiation may comprise broadband radiation, especially broadband radiation having one or more frequencies in the range of 1 - 250 kHz, especially in the range of 10 - 150 kHz. Hence, in further embodiments, the acoustic excitation radiation may be broadband acoustic excitation radiation having one or more frequencies in the range of 1 - 250 kHz.

In embodiments, the analysis stage may comprise the control system fitting at least part of the emission-related signal to a model of flexural modes of a cylindrical beam, and determining the physical vessel parameter based on the model.

In further embodiments, the analysis stage may comprise the control system fitting an exponential decay curve to at least part of the emission-related signal and determining a decay parameter, wherein the physical vessel parameter is determined based on the decay parameter, especially wherein the physical vessel parameter comprises one or more of a xylem vessel radius, a sap viscosity, and a xylem viscoelasticity.

In further embodiments, the analysis stage may comprise the control system determining one or more peaks in at least part of the emission-related signal in the frequency domain, wherein the physical vessel parameter is determined based on the one or more peaks, and especially wherein the physical vessel parameter comprises one or more of a xylem vessel element length, and a Young's modulus.

In embodiments, the operational mode may comprise an execution stage, wherein the execution stage comprises the control system controlling a plant cultivation condition (of the vascular plant) based on the physical vessel parameter, wherein the plant cultivation condition is selected from the group comprising a watering regime, a lighting regime, and a nutrient regime. Hence, in embodiments, the system may comprise or be functionally coupled to a plant cultivation system, especially wherein the plant cultivation system is configured to control one or more of (a) water, (b) lighting, and (c) nutrients provided to the vascular plant.

In embodiments, the excitation stage may comprise the acoustic radiation device providing an excitation frequency sweep from a first frequency to a second frequency. In further embodiments, the detection stage may comprise the acoustic radiation device providing an emission frequency sweep from the first frequency to the second frequency. In such embodiments, the first frequency and the second frequency may especially be selected from the range of 1-250 kHz.

In a further aspect, the invention may provide a computer program product comprising program instructions for execution on a control system functionally coupled to a system according to the invention, wherein the instructions, when executed by the control system, cause the system to carry out the method according to the invention.

In a further aspect, the invention may provide a data carrier carrying thereupon program instructions which, when executed by a control system functionally coupled to a system according to the invention cause the system to carry out the method according to the invention.

In a further aspect, the invention may provide a use of acoustic emission radiation from a vascular plant emitted by the vascular plant for determining a physical vessel parameter of a vascular tissue in the vascular plant, especially wherein the physical vessel parameter comprises an elasticity or a vessel dimension.

Hence, the invention may provide the use of acoustic emission radiation for determining a physical vessel parameter of a vascular tissue in a vascular plant, wherein the acoustic emission radiation is emitted by the vascular plant.

In embodiments, the acoustic emission radiation may have a frequency selected from the range of 1 - 250 kHz.

In further embodiments, the vascular plant, especially the vascular tissue, is exposed to acoustic excitation radiation (selected) such that the vascular plant emits acoustic emission radiation.

In further embodiments, the acoustic emission radiation is naturally emitted by the vascular plant.

In further embodiments, the vascular plant may be in a stressed condition. In particular, the vascular plant may naturally emit acoustic emission radiation in a stressed condition, such as due to water shortage. Hence, in further embodiments, the vascular plant may be in a stressed conditions as a result of drought.

In further embodiments, the use may comprise detecting the acoustic emission radiation with the system according to the invention. In further embodiments, the use may comprise detecting the acoustic emission radiation with an acoustic radiation device, more especially with a radiation detection device.

The term "stage" and similar terms used herein may refer to a (time) period (also "phase") of a method and/or an operational mode. The different stages may (partially) overlap (in time). For example, the excitation stage may, in general, be initiated prior to the detection stage, but may partially overlap in time therewith. However, for example, the detection stage may typically be completed prior to the analysis stage. It will be clear to the person skilled in the art how the stages may be beneficially arranged in time. For example, the excitation stage may (completely) occur prior to the detection stage such that the analysis stage may comprise an analysis of damping of the acoustic emission radiation in the time-domain. Hence, in embodiments, the excitation stage and the detection stage may be temporally separated.

The system, especially the control system, may have an operational mode. The term "operational mode" may also be indicated as "controlling mode". The system, or apparatus, or device (see further also below) may execute an action in a "mode" or "operational mode" or "mode of operation". Likewise, in a method an action, stage, or step may be executed in a "mode" or "operation mode" or "mode of operation". This does not exclude that the system, or apparatus, or device may also be adapted for providing another operational mode, or a plurality of other operational modes. Likewise, this does not exclude that before executing the mode and/or after executing the mode one or more other modes may be executed. However, in embodiments a control system (see further also below) may be available, that is adapted to provide at least the operational mode. Would other modes be available, the choice of such modes may especially be executed via a user interface, though other options, like executing a mode in dependence of a sensor signal or a (time) scheme, may also be possible. The operation mode may in embodiments also refer to a system, or apparatus, or device, that can only operate in a single operation mode (i.e. "on", without further tunability).

The term "controlling" and similar terms herein may especially refer at least to determining the behavior or supervising the running of an element. Hence, herein "controlling" and similar terms may e.g. refer to imposing behavior to the element (determining the behavior or supervising the running of an element), etc., such as e.g. measuring, displaying, actuating, opening, shifting, changing temperature, etc.. Beyond that, the term "controlling" and similar terms may additionally include monitoring. Hence, the term "controlling" and similar terms may include imposing behavior on an element and also imposing behavior on an element and monitoring the element. The controlling of the element can be done with a control system. The control system and the element may thus at least temporarily, or permanently, functionally be coupled. The element may comprise the control system. In embodiments, the control system and the element may not be physically coupled. Control can be done via wired and/or wireless control. The term "control system" may also refer to a plurality of different control systems, which especially are functionally coupled, and of which e.g. one control system may be a control system and one or more others may be slave control systems.

The embodiments described herein are not limited to a single aspect of the invention. For example, an embodiment describing the method may, for example, further relate to the system, especially to an operational mode of the system, or especially to the control system. Similarly, an embodiment of the system describing an operation of the system may further relate to embodiments of the method. In particular, an embodiment of the method describing an operation (of the system) may indicate that the system may, in embodiments, be configured for and/or be suitable for the operation.

BRIEF DESCRIPTION OF THE DRAWINGS

Embodiments of the invention will now be described, by way of example only, with reference to the accompanying schematic drawings in which corresponding reference symbols indicate corresponding parts, and in which: Fig.1 schematically depicts embodiments of the method and the system of the invention. Fig. 2 schematically depicts an a vascular tissue and a resonating beam model representation thereof. Fig. 3A-E schematically depict experimental data obtained using the method of the invention. The schematic drawings are not necessarily on scale.

DETAILED DESCRIPTION OF THE EMBODIMENTS

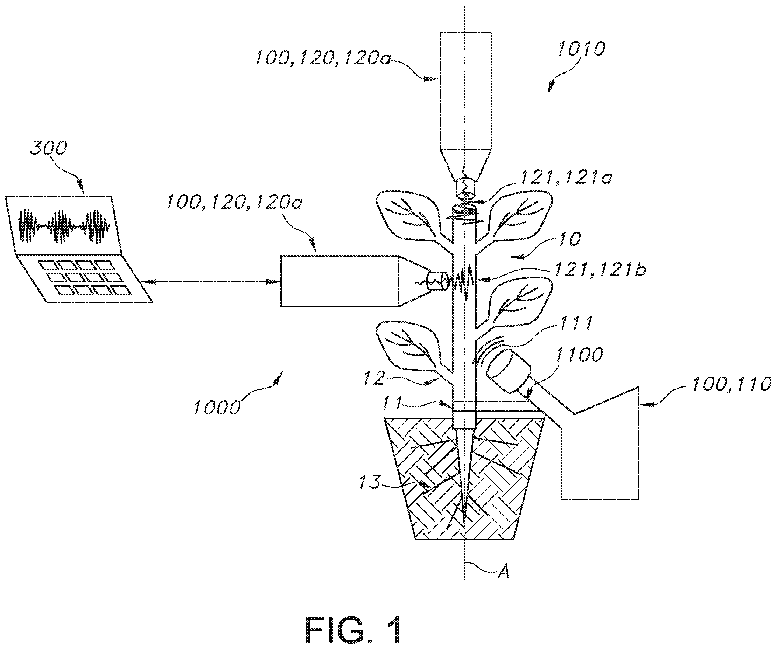

Fig. 1 schematically depicts the method and the system 1000 of the invention.

In particular, Fig. 1 schematically depicts the method for determining a physical vessel parameter of a vascular tissue 20 in a vascular plant 10. The method may comprise an excitation stage, a detection stage and/or an analysis stage.

The excitation stage may comprise providing acoustic excitation radiation 111 to the vascular plant 10, especially wherein the acoustic excitation radiation 111 comprises radiation having a frequency matching a resonance frequency of the vascular tissue 20, especially of a xylem tissue. In embodiments, the acoustic excitation radiation 111 may comprise radiation having a frequency selected from the range of 1 - 250 kHz.

The detection stage may comprise (resonance) acoustic emission radiation 121 from the vascular plant 10, especially from the vascular tissue 20. The detection stage may further comprise providing an emission-related signal.

The analysis stage may comprise determining the physical vessel parameter based on the emission-related signal, especially wherein the physical vessel parameter comprises a viscosity, an elasticity or a vessel dimension (of the vascular tissue 20), especially an elasticity or a vessel dimension.

In embodiments, the vascular tissue 20 may be a xylem tissue in a plant stem 11, in a plant branch 12, or in a plant root 13.

The vascular tissue 20 may have a longitudinal axis A. In particular, the longitudinal axis A may be parallel to a longitudinal axis of the plant stem 11. In the depicted embodiment, the detection stage comprises: acoustic emission radiation 121 from the vascular plant 10 at a first location arranged axially with respect to the longitudinal axis A; and acoustic emission radiation 121 from the vascular plant at a second location arranged perpendicular with respect to the longitudinal axis A.

In further embodiments, one or more applies of: the excitation stage comprising an excitation frequency sweep from a first frequency to a second frequency; and the detection stage comprising an emission frequency sweep from the first frequency to the second frequency; Especially wherein the first frequency and the second frequency are selected from the range of 1-250 kHz.

In further embodiments, the method may comprise determining the physical vessel parameter for a plurality of vascular plants 10, wherein the method comprises selecting one or more vascular plants 10 of the plurality of vascular plants 10 for breeding based on the physical vessel parameters.

Fig. 1 further schematically depicts a system 1000 for determining a physical vessel parameter of a vascular tissue 20 in a vascular plant 10, wherein the system 100 comprises an acoustic radiation device 100 and a control system 300. The acoustic radiation device may especially comprise one or more of a radiation generation device 110 and a radiation detection device 120, especially an ultrasound generator and an ultrasound sensor. The system 1000, especially the control system 300, may have an operational mode. The operational mode may especially comprise one or more of an excitation stage, a detection stage and an analysis stage.

In the excitation stage, the radiation generation device 110 may be configured to provide acoustic excitation radiation 111 to the vascular plant 10, especially to the vascular tissue 20. Hence, the excitation stage may comprise the acoustic radiation device 100, especially the radiation generation device 110, providing acoustic excitation radiation 111 to the vascular plant 10, especially to the vascular tissue 20. The acoustic excitation radiation 111 may comprise radiation having a frequency matching a resonance frequency of the vascular tissue 20, especially of a xylem tissue. In particular, the acoustic excitation radiation 111 may comprise radiation having a frequency selected from the range of 1-250 kHz.

In the detection stage, the radiation detection device 120 may be configured to detect (resonance) acoustic emission radiation 121 from the vascular plant, especially from the vascular tissue 20, and to provide an emission-related signal to the control system 300. Hence, the detection stage may comprise the acoustic radiation device 100, especially the radiation detection device 120, detecting (resonance) acoustic emission radiation 121 from the vascular plant 10 and providing an emission-related signal to the control system 300.

In the analysis stage, the control system may be configured to determine the physical vessel parameter based on the emission-related signal, wherein the physical vessel parameter comprises an elasticity or a vessel dimension. Hence, the analysis stage may comprise the control system 300 determining the physical vessel parameter based on the emission-related signal, wherein the physical vessel parameter comprises an elasticity or a vessel dimension.

In the depicted embodiment, the acoustic radiation device 100 is physically coupled to the control system 300. In further embodiments, the acoustic radiation device 100 may comprise a transmitter, wherein the transmitter is configured to (wirelessly) provide the emission-related signal to the control system 300.

In embodiments, the system 1000 may comprise a stem mount 1100 configured for attaching the acoustic radiation device 100, especially the radiation generation device 110, or especially the radiation detection device 120, to a plant stem 11 of the vascular plant 10. In such embodiments, the operational mode may comprise providing acoustic excitation radiation 111 to the plant stem 11 and detecting acoustic emission radiation from the plant stem 11. In the depicted embodiment, the system 1000 comprises a stem mount 1100 configured for attaching the radiation generating device 110 to the plant stem 11.

The vascular tissue 20, especially the xylem tissue, may have a longitudinal axis A, which may, for example, be parallel to a longitudinal axis of the plant stem 11 (or the plant branch 12). In embodiments, the acoustic radiation device 100 may comprise an axial radiation detection device 120,120a and/or a radial radiation detection device 120,120b, especially an axial radiation detection device 120,120a, or especially a radial radiation detection device 120,120b. In such embodiments, in the operational mode: the axial radiation detection device 120,120a may be arranged at a first location arranged axially along the longitudinal axis A; and/or the radial radiation detection device 120,120b may be arranged at a second location arranged perpendicular to the longitudinal axis A.

In embodiments, the system 1000 may further comprise a measurement site 1010 configured for hosting the vascular plant 10, wherein: the acoustic radiation device 100 comprises an axial radiation detection device 120,120a arranged at a first location (in the measurement site 1010) arranged axially along the longitudinal axis A (of the hosted vascular plant 10); and/or the acoustic radiation device 100 comprises a radial radiation detection device 120,120b arranged at a second location arranged perpendicular to the longitudinal axis A (of the hosted vascular plant 10).

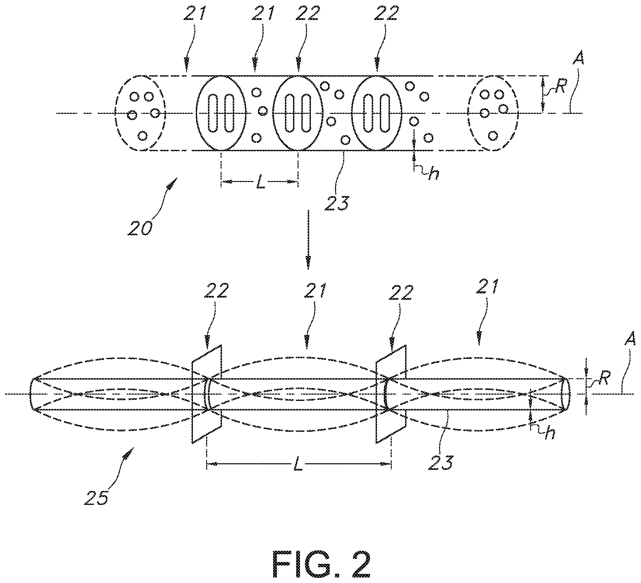

Fig. 2 schematically depicts a vascular tissue 20, especially a xylem tissue, and a resonating beam model representation 25 thereof. The vascular tissue 20 may comprise a plurality of vascular tissue elements 21, especially xylem vessel elements, separated by perforation plates 22. Hence, a xylem vessel element may be defined by (the presence of) perforation plates 22 at each end. The xylem vessel elements may define the resonating unit for the ultrasound. The vascular tissue 20, especially the vascular tissue element 21 may have a radius R. Further, the vascular tissue element 21, especially the xylem vessel element, may have a length L, especially a xylem vessel length L. The vascular tissue 20 may further comprise a vascular tissue wall 23, wherein the vascular tissue wall 23 may have a wall thickness h, especially a xylem wall thickness h.

In embodiments, the analysis stage may comprise fitting at least part of the emission-related signal to a model of flexural modes of a cylindrical beam, and determining the physical vessel parameter (at least partially) based on the model. The dashed lines in Fig. 2 schematically depict the instantaneous shape of a vibrating beam.

## Experiments

Experiments were performed based on naturally emitted ultrasound radiation by plants. In particular, during water-shortage in the roots and/or heavy transpiration, a water column in the xylem may be subjected to a large tensile stress. Beyond a critical tension, water may exist in a metastable state for a short time, which may lead to local cavitation. The stress may be released via gas-bubble nucleation. Apart from cavitation, bubble formation inside the xylem can occur through "air-seeding", due to a pressure difference across the air-water meniscus at pores on xylem cell walls. The process of bubble formation may result in a release of the elastic energy stored in the water column, a part of which may be converted to acoustic emission radiation. In particular, peak frequencies may be observed across a multitude of (ultra-)sound pulses in the range 1-250 kHz, especially in the range of 10-150 kHz, which may be much lower than the theoretical resonant bubble frequencies. Hence, experiments were performed to determine whether this acoustic emission radiation is indicative of physical vessel parameters, especially a physical vessel parameter of a xylem tissue. The physical vessel parameter was also determined using prior art methods as comparative examples.

Hence, a vascular plant 10 may (spontaneously) emit acoustic emission radiation 121, especially from the vascular tissue 20, which may be indicative of a physical vessel parameter. However, in embodiments, the method may especially comprise providing acoustic excitation radiation 111 to the vascular plant 10, which may then subsequently provide the acoustic emission radiation 121.

## Methods

Sample collection. Three potted biological replicates of *Hydrangea quercifolia L.* were obtained from "Intratuin" garden center (outdoor) in Elst, the Netherlands (51.913o N , 5.87o E) on 19th November 2019 at 0930 hrs, and moved to the indoor laboratory environment by 1030 hrs on the same day at the Wageningen University and Research. Three leafy shoot samples labelled A, B, and C, one from each potted plant, were cut keeping the leaves intact and immediately kept in tap water to prevent embolism in the xylem vessels at the cut-end. From each sample, a 60-70 mm long trimmed (without leaf-petioles) stem segment was cut inside water to reduce, especially prevent, air entry and blockage. The segments were approximately cylindrical with a cross-sectional diameter of ~ 5-6 mm.

Recording ultrasound emission. The leafy shoot samples A, B and C were then taken out of water, the excess water wiped from the stem-surface using tissue paper, and left on the bench for air-drying, resulting in an accelerated drought stress. A M500-USB ultrasound microphone (with a reliable sensitivity window between 10 kHz and 150 kHz) from Pettersson was placed first in the axial (-2 mm from the cut-face of stem normal to the cross-section) and then in the radial (on the cylindrical surface of the stem) directions to record acoustic emission radiation at a sampling rate of 500 kHz in continuous time windows of 120 seconds. The frequency spectra of the measured signals were obtained via a 250-point Discrete Fourier Transform, spanning a time frame of 1.5 ms.

Comparative example - latex-paint infusion technique as described in Chatelet et al., "Xylem Structure and Connectivity in Grapevine (Vitis vinifera) Shoots Provides a Passive Mechanism for the Spread of Bacteria in Grape Plants", Annals of Botany, 2006, which is hereby herein incorporated by reference. The stem segments were mounted vertically over a glass container filled with degassed latex-paint solution, with one end immersed in the paint and the other end tightly inserted into a plastic tube connected to a suction pump that applies a pressure difference of 400 mbar. The stem-tube junction was taped and smeared with Vaseline to prevent air leakage. As the pump sucked the solution through the stem for a duration of 12 hours, the paint filled up and remained confined in one xylem vessel while the clear water was conducted through the entire stem (via the bordering pits between adjacent xylem vessels) and emerged out in the tube. Subsequently, the stem samples were sliced with a blade at intervals of 5 mm. The number of painted vessels were then counted on each face of the cut slices from images captured by a VHX digital microscope from Keyence.

Comparative example - Scanning electron cryo-microscopy (cryo-SEM) of xylem wall and vessel elements. Transverse and longitudinal cross-sections of randomly chosen hydrangea stem segments were made using a razorblade. The cross-sections were left on filter paper for 1-2 minutes to remove most of the adhering water. After that the sections were fixed to a sample holder using Tissue-Tek. The samples were frozen by plunging the sample holder into liquid nitrogen. Subsequently the samples were transferred to a cryo-preparation chamber (Leica) under vacuum where it was kept at -90°C for 3 minutes to remove ice from the surface (freeze etching to remove water vapor contamination). Still under vacuum the samples were coated with 12 nm of tungsten and transferred using a VCT100 shuttle (Leica) to a field emission scanning electron microscope (Magellan 400 from FEI). The samples were analyzed at 2 kV, 13 pA at - 120°C. The physical thickness of the xylem cell walls was observed to be ~1 $\mu$m. The length of individual xylem vessel elements were observed to be between 0.6 mm and 1 mm.

Comparative example - uniaxial tensile loading for elastic modulus determination. Multiple stem segments of lengths in the range of 4-7 cm were cut and mounted vertically between two clamps of a Zwick/Roell Z005 tensile testing machine. The initial pre-strained length is equal to the vertical separation between the clamps and was kept as 20 mm. The uniaxial stress is calculated as the tensile force applied by the equipment divided by the average cross-section area of the stem segment, and the longitudinal strain is calculated as the change in stem length per unit initial length. The Young's modulus E is then extracted as the slope of the linear part of the stress-strain curve at small values of strain (about $10^{-4}$ ). The average mass density of each sample was also calculated from measured weight and volume just before the tensile loading procedure. The weights were measured with a Scaltec SBC 33 precision balance, while the dimensions were measured with a Vernier Caliper with a resolution of 0.1 mm.

**Measurements**

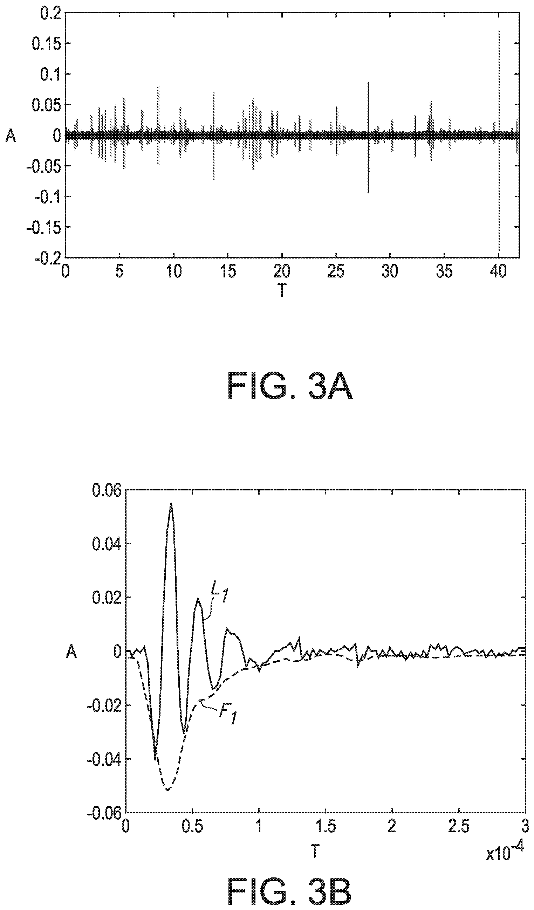

Ultrasound measurements. Freshly cut and hydrated stem samples were placed on a bench to induce accelerated drought stress at room temperature during daytime. Ultrasound pulses were measured with a broadband ultrasound microphone placed at a first location arranged axially with respect to a longitudinal axis A of the plant stem 11, and at a second location arranged perpendicular with respect to the longitudinal axis A of the plant stem 11. Fig. 3A depicts ultrasound emissions recorded at the first location and starting after 5 minutes into the drying process in amplitude A (in a.u.) versus time T (in seconds).

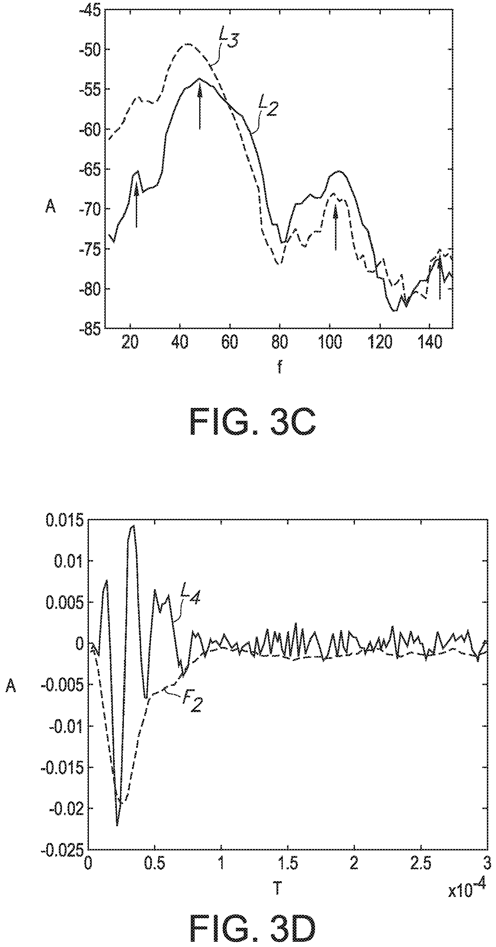

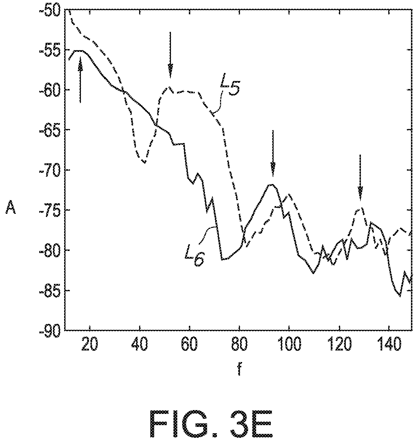

Fig. 3B and Fig. 3D schematically depict representative time-domain waveforms of single pulses for sample C in pulse amplitude A (in a.u.) over time T (in seconds) at the first location (Fig. 3B) and at the second location (Fig. 3D). Fig. 3C and 3E schematically depict corresponding frequency domain spectra in pulse amplitude A (in dB) versus frequency f (in kHz) (obtained via a Fourier transformation of the pulse in time domain), wherein the 0 dB line refers to an amplitude of 1 in the time domain.

Fig. 3B depicts observations obtained at the first location. Specifically, Fig. 3B depicts line $L_1$ representing a pulse starting at T=33.7263 s, and a corresponding fit envelope Fi. The envelope of the pulse amplitude in time-domain decays exponentially with a 1/e time constant $\tau_s$, wherein $\tau_s$ specifically refers to an acoustic emission radiation settling time. The (acoustic emission radiation) settling time $\tau_s$ of the pulse can be obtained by fitting an exponential function to the fit envelope according to the formula

$$A(t) = A_0 * e^{-T/\tau_s}$$

wherein $A_0$ is the amplitude at the peak of the pulse (here at about T=0.3s).

Fig. 3C schematically depicts frequency spectra corresponding to exemplary pulses measured at the first location. Specifically, line $L_2$ corresponds to a pulse starting at T=19.5648 s and line $L_3$ corresponds to a pulse starting at T=33.7263 s. The arrows indicate (broad) peaks that were used to determine physical vessel parameters (see below).

Fig. 3D depicts observations obtained at the second location. Specifically, Fig. 3D depicts line $L_4$ representing a pulse starting at T=72.5614 s, and a corresponding fit envelope $F_2$.

Fig. 3E schematically depicts frequency spectra corresponding to exemplary pulses measured at the second location. Specifically, line $L_5$ corresponds to a pulse starting at T=48.4442 s and line $L_6$ corresponds to a pulse starting at T=99.1434 s. The arrows indicate (broad) peaks that were used to determine physical vessel parameters (see below).

Based on the experimental measurements, the most probable $\tau_s$= 19.1 μs, 20.9 μs, and 20 μs, for samples A, B, and C, respectively, for the pulses in the axial direction (measured at the first location). The corresponding values $\tau_s$ for the radial direction (measured at the second location) were 33.8 μs, 14.1 μs, and 21 μs.

The most probable peak frequency $f_p$ (axial) with the largest amplitude in the sound pulses recorded axially from sample A, B, and C were found to be 36.5±4 kHz, 32.7±5 kHz, and 42.3±8.5 kHz respectively. In addition, peaks close to integral multiples of $f_p$ (axial) are observed as shown in Fig. 3C. The observed and calculated frequencies for the first location are summarized in table 1 below:

| Mode | Sample A | | Sample B | | Sample C | |
|---|---|---|---|---|---|---|
| m | Observed [kHz] | Calculated [kHz] | Observed [kHz] | Calculated [kHz] | Observed [kHz] | Calculated [kHz] |
| 1 | 30-40 | 35 | 32-38 | 36 | 40-50 | 46 |
| 2 | 60-70 | 70 | 68-72 | 72 | 90-110 | 92 |
| 3 | 100-120 | 105 | 96-115 | 108 | 140-150 | 138 |
| 4 | 135-140 | 140 | 128-145 | 144 | Below noise floor | ---- |

In table 1, the observed values refer to experimental measurements, whereas the calculated values are obtained by assuming that the observed frequency in the 30-50 kHz range corresponds to m=1, i.e., assuming that the smallest observed frequencies correspond to a mode order of 1.

Hence, as the observed values lie close to the calculated values, the resonance frequencies may be accurately determined from the acoustic emission radiation measured at the first location.

A similar behavior was observed in the frequency spectra of the radially recorded ultrasound pulses (see Fig. 3E), with the lowest characteristic peak frequency in the range 15 - 20 kHz present in all the three samples. In addition, characteristic frequencies corresponding to higher order resonances were observed mostly in the following intervals: 30-40 kHz, 55-70 kHz, 84-92 kHz, and 110-140 kHz. The observed and calculated frequencies for the second location are summarized in table 2 below:

| Mode | | Sample A | | Sample B | | Sample C | |
|---|---|---|---|---|---|---|---|
| n | $k_T$ | Observed [kHz] | Calculated [kHz] | Observed [kHz] | Calculated [kHz] | Observed [kHz] | Calculated [kHz] |
| 1 | 4.73 | - | 5.4-7.25 | - | 8 | - | 5.4-6.5 |
| 2 | 7.8532 | 15-20 | 15-20 | 18-24 | 22 | 15-18 | 15-18 |
| 3 | 10.996 | 30-40 | 29.4-39.2 | 32-46 | 43.1 | 30-35 | 29.4-35.2 |
| 4 | 14.137 | 55-70 | 48.6-64.8 | 63-75 | 71.24 | 48-52 | 48.7-58.3 |
| 5 | 17.137 | 84-92 | 72.6-96.8 | 90-110 | 106.4 | 90-100 | 72.8-87.2 |
| 6 | 20.42 | 110-120 | 101.4-135.2 | 126-142 | 148.63 | 110-135 | 101.6-121.8 |

In table 2, the observed values refer to experimental measurements, whereas the calculated values are obtained by assuming that the observed frequency in the 15-24 kHz range corresponds to n=2, i.e., assuming that the smallest observed frequencies correspond to a mode order of 2.

Hence, similarly as for the measurements at the first location, the resonance frequencies may also be accurately determined from the acoustic emission radiation measured at the second location.

The acoustic emission radiation observed at the first location was interpreted by modelling the xylem vessel as a cylindrical pipe of effective length L sustaining longitudinal standing waves in the water whose resonance frequencies depend on the mode order m, and the longitudinal speed of sound in the pipe $v_{eff}$. These sound waves (expected to be dominant in the axially recorded ultrasound) will undergo damping primarily due to the dynamic viscosity $\eta_1$ in the xylem. The damped oscillations can be described by a 2nd order linear resonator where the damping ratio $\zeta$ is a function of the acoustic inductance, resistance and capacitance of the system. From the observed $f_p$(axial) and $\tau_s$, $\zeta$ is obtained as

$$\zeta = \frac{1}{\sqrt{1 + \left(f_{p(axial)} \cdot \tau_s\right)^2}}$$

and the effective acoustic xylem radius R is obtained as:

$$R = \sqrt{\frac{4 \cdot \eta_1 \cdot \tau_s}{\rho_l}}$$

where $\rho l$ is the mass density of sap (water). Note that in this model, R can calculated independently of the length L, from the settling time of the measured time-domain acoustic signal.

The resonance frequency $f_L$ can be calculated from $f_p$ (axial) and $\zeta$ using:

$$f_p = f_L \sqrt{1 - \zeta^2}$$

The effective xylem length L, especially the length L of a xylem vessel element 21 can be expressed as:

$$\frac{1}{L^2} = \frac{4 f_L^2}{m^2 v_{eff}^2} = \frac{4 f_L^2}{m^2} \left[ \frac{1}{v_l^2} + \frac{2 \rho_l R}{h} \cdot \left(\frac{1}{E}\right) \right]$$

Where vi is the speed of sound in bulk water ($\approx$ 1485 m/s at 20 °C), E is the elastic modulus of the xylem wall and h is the effective wall thickness (a fit parameter).

Hence, in embodiments, the method, especially the analysis stage, may comprise fitting an exponential decay curve to at least part of the emission-related signal (in the time domain) and determining a decay parameter, wherein the physical vessel parameter is determined based on the decay parameter, especially wherein the physical vessel parameter comprises one or more of a xylem vessel radius R, a sap viscosity, and a xylem viscoelasticity.

In further embodiments, the method, especially the analysis stage, may comprise determining one or more peaks in at least part of the emission-related signal in the frequency domain, wherein the physical vessel parameter is determined based on the one or more peaks, and wherein the physical vessel parameter comprises one or more of a xylem vessel element length L, and a Young's modulus E.

In embodiments, the method, especially the analysis stage, may comprise fitting at least part of the emission-related signal to a model of flexural modes of a cylindrical beam, and determining the physical vessel parameter based on the model. In particular, the acoustic emission radiation observed at the second location was analyzed with a model of flexural modes of a cylindrical beam surrounded by a viscoelastic material. A good match for the set of frequencies was obtained if the 2nd order mode is assigned to the frequency range 15-20 kHz for all the samples. Essentially, the xylem vessels are modelled as a viscoelastic cylindrical beam: a cell wall - water composite with an effective mass density $\rho_{xylem}$. Freshly cut stem segments, collected from the same plant 10 and similar to the ones used for sound recording and paint infusion, were used to measure a mean mass density of $\approx$1300 $\pm$ 300 kg.m$^{-3}$ which in turn provides a close estimate of $\rho_{xylem}$. Using the beam model, this may result in:

$$\frac{1}{L^2} = \frac{\left(4 \pi f_T \sqrt{\rho_{xylem}}\right)}{k_T^2 R} \cdot \left(\frac{1}{\sqrt{E}}\right)$$

Where $f_T$ is the resonance frequency of transverse vibrations and $k_T$ is the mode constant. In combination with the equations above, this provides:

$$E = \left[ \frac{4\rho_l R^2 k_T^2 f_L^2 v_l}{(m^2 \pi h f_T \rho_{xylem}^{\frac{1}{2}} v_l) + \sqrt{m^4 \pi^2 h^2 f_T^2 \rho_{xylem} v_l^2 - 8h\rho_l R^3 k_T^4 f_L^4}} \right]^2$$

The time-domain damping in the radially recorded ultrasound may be dominated by the viscosity ($\eta_{solid}$) in the solid matter comprising the cell wall, xylem fibres, cambium and other vascular tissues. Such a system may be modeled as a linear Maxwell material and $\eta_{solid}$ may be obtained from the 1/e settling time $\tau_s$ of the pulse amplitude as:

$$\tau_s = \frac{\eta_{solid}}{E}$$

Various physical vessel parameters were determined using the method of the invention - using above-mentioned equations - as well as using comparative (destructive) methods. The determined values are summarized in table 3 below:

| Parameter | Based on acoustic emission radiation | Comparative method | |
|---|---|---|---|
| R [μm] | 9.89 ± 1.6 | 9- 18 | Optical microscopy; scanning electron microscopy |
| Xylem vessel element length L [μm] | 1.08 ± 0.18 | 6.38 - 8.45 | Scanning electron microscopy |
| E [GPa] | 0.4 ± 0.1 | 0.6 - 1.0 | Uniaxial tensile loading |
| $\eta_{solid}$ [kPa.s] | 10.8 ± 2.3 | - | - |
| h [μm] | 0.8 | 1.0 | Scanning electron microscopy |

Hence, the parameters may be accurately determined using non-destructive observations of acoustic emission radiation.

In particular, the experiments may demonstrate the use of acoustic emission radiation from a vascular plant emitted by the vascular plant for determining a physical vessel parameter of a vascular tissue in the vascular plant.

The term "plurality" refers to two or more. Furthermore, the terms "a plurality of" and "a number of" may be used interchangeably.

The terms "substantially" or "essentially" herein, and similar terms, will be understood by the person skilled in the art. The terms "substantially" or "essentially" may also include embodiments with "entirely", "completely", "all", etc. Hence, in embodiments the adjective substantially or essentially may also be removed. Where applicable, the term "substantially" or the term "essentially" may also relate to 90% or higher, such as 95% or higher, especially 99% or higher, even more especially 99.5% or higher, including 100%. Moreover, the terms "about" and "approximately" may also relate to 90% or higher, such as 95% or higher, especially 99% or higher, even more especially 99.5% or higher, including 100%. For numerical values it is to be understood that the terms "substantially", "essentially", "about", and "approximately" may also relate to the range of 90% - 110%, such as 95%-105%, especially 99%-101% of the values(s) it refers to.

The term "comprise" includes also embodiments wherein the term "comprises" means "consists of".

The term "and/or" especially relates to one or more of the items mentioned before and after "and/or". For instance, a phrase "item 1 and/or item 2" and similar phrases may relate to one or more of item 1 and item 2. The term "comprising" may in an embodiment refer to "consisting of" but may in another embodiment also refer to "containing at least the defined species and optionally one or more other species".

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

The devices, apparatus, or systems may herein amongst others be described during operation. As will be clear to the person skilled in the art, the invention is not limited to methods of operation, or devices, apparatus, or systems in operation.

The term "further embodiment" and similar terms may refer to an embodiment comprising the features of the previously discussed embodiment, but may also refer to an alternative embodiment.

It should be noted that the above-mentioned embodiments illustrate rather than limit the invention, and that those skilled in the art will be able to design many alternative embodiments without departing from the scope of the appended claims.

In the claims, any reference signs placed between parentheses shall not be construed as limiting the claim.

Use of the verb "to comprise" and its conjugations does not exclude the presence of elements or steps other than those stated in a claim. Unless the context clearly requires otherwise, throughout the description and the claims, the words "comprise", "comprising", "include", "including", "contain", "containing" and the like are to be construed in an inclusive sense as opposed to an exclusive or exhaustive sense; that is to say, in the sense of "including, but not limited to".

The article "a" or "an" preceding an element does not exclude the presence of a plurality of such elements.

The invention may be implemented by means of hardware comprising several distinct elements, and by means of a suitably programmed computer. In a device claim, or an apparatus claim, or a system claim, enumerating several means, several of these means may be embodied by one and the same item of hardware. The mere fact that certain measures are recited in mutually different dependent claims does not indicate that a combination of these measures cannot be used to advantage.

The invention further applies to a device, apparatus, or system comprising one or more of the characterizing features described in the description and/or shown in the attached drawings. The invention further pertains to a method or process comprising one or more of the characterizing features described in the description and/or shown in the attached drawings. Moreover, if a method or an embodiment of the method is described being executed in a device, apparatus, or system, it will be understood that the device, apparatus, or system is suitable for or configured for (executing) the method or the embodiment of the method respectively.

The various aspects discussed in this patent can be combined in order to provide additional advantages. Further, the person skilled in the art will understand that embodiments can be combined, and that also more than two embodiments can be combined. Furthermore, some of the features can form the basis for one or more divisional applications.

**Claims**

1. A method for determining a physical vessel parameter of a vascular tissue (20) in a vascular plant (10), wherein the method comprises:

   - a detection stage comprising detecting acoustic emission radiation (121) from the vascular plant (10) and providing an emission-related signal; and
   - an analysis stage comprising determining the physical vessel parameter based on the emission-related signal, wherein the physical vessel parameter comprises an elasticity or a vessel dimension, and wherein the analysis stage comprises fitting at least part of the emission-related signal to a model of flexural modes of a cylindrical beam, and determining the physical vessel parameter based on the model.

2. The method according to any one of the preceding claims, wherein the method comprises:

   - an excitation stage comprising providing acoustic excitation radiation (111) to the vascular plant (10), wherein the acoustic excitation radiation (111) comprises radiation having a frequency selected from the range of 1 - 250 kHz, and optionally wherein the acoustic excitation radiation (111) comprises broadband radiation having one or more frequencies in the range of 10 - 150 kHz.

3. The method according to claim 2, wherein the excitation stage comprises providing the acoustic excitation radiation (111) via a pulse, wherein one or more applies of:

   - the pulse has a pulse duration that is larger than a characteristic settling time due to damping of the vascular tissue (20); and
   - the pulse has a pulse duration smaller than a time-of-flight of the acoustic excitation radiation through the vascular tissue (20).

4. The method according to any one of the preceding claims 2-3, wherein one or more applies of:

   - the excitation stage comprises an excitation frequency sweep from a first frequency to a second frequency; and
   - the detection stage comprises an emission frequency sweep from the first frequency to the second frequency;

wherein the first frequency and the second frequency are selected from the range of 1-250 kHz.

5. The method according to any one of the preceding claims, wherein the vascular tissue (20) has a longitudinal axis (A), and wherein the detection stage comprises detecting:

   - acoustic emission radiation (121) from the vascular plant (10) at a first location arranged axially with respect to the longitudinal axis (A); and/or
   - acoustic emission radiation (121) from the vascular plant (10) at a second location arranged perpendicular with respect to the longitudinal axis (A).

6. The method according to any one of the preceding claims, wherein the analysis stage comprises one or more of the following:

   - fitting an exponential decay curve to at least part of the emission-related signal and determining a decay parameter, wherein the physical vessel parameter is determined based on the decay parameter, wherein the physical vessel parameter comprises one or more of a xylem vessel radius, a sap viscosity, and a xylem viscoelasticity; and
   - determining one or more peaks in at least part of the emission-related signal in the frequency domain, wherein the physical vessel parameter is determined based on the one or more peaks, and wherein the physical vessel parameter comprises one or more of a xylem vessel element length, and a Young's modulus.

7. The method according to any one of the preceding claims, wherein the method further comprises one or more of the following:

   - controlling a plant cultivation condition based on the physical vessel parameter, wherein the plant cultivation condition is selected from the group comprising a watering regime, a lighting regime, and a nutrient regime; and
   - determining the physical vessel parameter for a plurality of vascular plants (10), wherein the method comprises selecting one or more vascular plants (10) of the plurality of vascular plants (10) for breeding based on the physical vessel parameters.

8. The method according to any one of the preceding claims, wherein one or more applies of:

   - the acoustic emission radiation (121) is naturally emitted by the vascular plant (10);
   - the vascular plant (10) is in a stressed condition;
   - the vascular plant (10) is in a stressed condition as a result of drought; and
   - the vascular tissue (20) is a xylem tissue in a plant stem (11) or in a plant branch (12).

9. A system (1000) for determining a physical vessel parameter of a vascular tissue (20) in a vascular plant (10), wherein the system (100) comprises an acoustic radiation device (100) and a control system (300), wherein the system (1000) has an operational mode, wherein the operational mode comprises:

   - a detection stage comprising the acoustic radiation device (100) detecting acoustic emission radiation (121) from the vascular plant (10) and providing an emission-related signal to the control system (300); and
   - an analysis stage comprising the control system (300) determining the physical vessel parameter based on the emission-related signal, wherein the physical vessel parameter comprises an elasticity or a vessel dimension, and the control system (300) fitting at least part of the emission-related signal to a model of flexural modes of a cylindrical beam, and determining the physical vessel parameter based on the model.

10. The system (1000) according to claim 9, wherein the operational mode further comprises one or more of the following:

    - an excitation stage comprising the acoustic radiation device (100) providing acoustic excitation radiation (111) to the vascular plant (10), wherein the acoustic excitation radiation comprises radiation having a frequency selected from the range of 1-250 kHz, optionally wherein the acoustic excitation radiation comprises broadband radiation having one or more frequencies in the range of 10 - 150 kHz; and
    - an execution stage, wherein the execution stage comprises the control system controlling a plant cultivation condition based on the physical vessel parameter, wherein the plant cultivation condition is selected from the group comprising a watering regime, a lighting regime, and a nutrient regime.

**11.** The system (1000) according to claim 10, wherein the system (1000) comprises a stem mount (1100) configured for attaching the acoustic radiation device (100) to a plant stem (11) of the vascular plant (10), wherein the operational mode comprises providing acoustic excitation radiation (111) to the plant stem (11) and detecting acoustic emission radiation (121) from the plant stem (11).

**12.** The system (1000) according to any one of the preceding claims 10-11, wherein the excitation stage comprises the acoustic radiation device (100) providing the acoustic excitation radiation (111) via a pulse, wherein one or more applies of:

- the pulse has a pulse duration that is larger than a characteristic settling time of the vascular tissue (20); and
- the pulse has a pulse duration smaller than a time-of-flight of the acoustic excitation radiation through the vascular tissue (20).

**13.** The system (1000) according to any one of the preceding claims 10-12, wherein:

- the excitation stage comprises the acoustic radiation device (100) providing an excitation frequency sweep from a first frequency to a second frequency; and
- the detection stage comprises the acoustic radiation device (100) providing an emission frequency sweep from the first frequency to the second frequency;

wherein the first frequency and the second frequency are selected from the range of 1-250 kHz.

**14.** The system (1000) according to claim 9-13, wherein the vascular tissue (20) has a longitudinal axis (A), and wherein the system (1000) further comprises a measurement site (1010) configured for hosting the vascular plant (10), wherein:

- the acoustic radiation device (100) comprises an axial radiation detection device radiation detection device (120a) arranged at a first location arranged axially along the longitudinal axis (A); and/or
- the acoustic radiation device (100) comprises a radial radiation detection device (120b) arranged at a second location arranged perpendicular to the longitudinal axis (A).

**15.** The system (1000) according to any one of the preceding claims 9-14, wherein the analysis stage comprises one or more of:

- the control system (300) fitting an exponential decay curve to at least part of the emission-related signal and determining a decay parameter, wherein the physical vessel parameter is determined based on the decay parameter, wherein the physical vessel parameter comprises one or more of a xylem vessel radius, a sap viscosity, and a xylem viscoelasticity; and
- the control system (300) determining one or more peaks in at least part of the emission-related signal in the frequency domain, wherein the physical vessel parameter is determined based on the one or more peaks, and wherein the physical vessel parameter comprises one or more of a xylem vessel element length, and a Young's modulus.

**Patentansprüche**

**1.** Verfahren zum Bestimmen eines physikalischen Gefäßparameters von Gefäßgewebe (20) in einer Gefäßpflanze (10), wobei das Verfahren umfasst:

- eine Detektionsstufe, die umfasst, akustische Emissionsausstrahlung (121) von der Gefäßpflanze (10) zu erfassen und ein emissionsbezogenes Signal bereitzustellen; und
- eine Analysestufe, die umfasst, den physikalischen Gefäßparameter basierend auf dem emissionsbezogenen Signal zu bestimmen, wobei der physikalische Gefäßparameter eine Elastizität oder eine Gefäßdimension umfasst, und wobei die Analysestufe umfasst, wenigstens einen Teil des emissionsbezogenen Signals an ein Modell von Biegemoden eines zylindrischen Strahls anzupassen und den physikalischen Gefäßparameter basierend auf dem Modell zu bestimmen.

**2.** Verfahren gemäß einem der vorstehenden Ansprüche, wobei das Verfahren umfasst:

- eine Erregungsstufe, umfassend, die Gefäßpflanze (10) mit akustischer Erregungsstrahlung (111) zu beaufschlagen, wobei die akustische Erregungsstrahlung (111) eine Strahlung mit einer Frequenz umfasst, die aus einem Bereich von 1-250 kHz ausgewählt ist, und wobei optional die akustische Erregungsstrahlung (111) breitbandige Strahlung mit einer oder mehreren Frequenzen im Bereich von 10-150 kHz umfasst.

3. Verfahren gemäß Anspruch 2, wobei die Erregungsstufe umfasst, die akustische Erregungsstrahlung (111) über einen Impuls bereitzustellen, wobei eines oder mehrere der folgenden gelten:

- der Impuls hat eine Impulsdauer, die größer ist als eine charakteristische Einschwingzeit aufgrund der Dämpfung des Gefäßgewebes (20); und
- der Impuls hat eine Impulsdauer, die kleiner ist als die Laufzeit der akustischen Erregungsstrahlung durch das Gefäßgewebe (20).

4. Verfahren gemäß einem der vorstehenden Ansprüche 2-3, wobei eines oder mehrere der folgenden gelten:

- die Erregungsstufe umfasst einen Erregungsfrequenzhub von einer ersten Frequenz zu einer zweiten Frequenz; und
- die Detektionsstufe umfasst einen Emissionsfrequenzhub von der ersten Frequenz zur zweiten Frequenz;

wobei die erste Frequenz und die zweite Frequenz aus dem Bereich von 1-250 kHz ausgewählt sind.

5. Verfahren gemäß einem der vorstehenden Ansprüche, wobei das Gefäßgewebe (20) eine Längsachse (A) aufweist und wobei die Detektionsstufe umfasst, folgendes zu detektieren:

- akustische Emissionsausstrahlung (121) von der Gefäßpflanze (10) an einer ersten, in Bezug zur Längsachse (A) axial angeordneten Stelle; und/oder
- akustische Emissionsausstrahlung (121) von der Gefäßpflanze (10) an einer zweiten, in Bezug zur Längsachse (A) senkrecht angeordneten Stelle.

6. Verfahren gemäß einem der vorstehenden Ansprüche, wobei die Analysestufe eines oder mehrere der folgenden umfasst:

- Anpassen einer exponentiellen Abklingkurve an wenigstens einen Teil des emissionsbezogenen Signals und Bestimmen eines Abklingparameters, wobei der physikalische Gefäßparameter basierend auf dem Abklingparameter bestimmt wird, wobei der physikalische Gefäßparameter eines oder mehrere von einem Xylemgefäßradius, einer Saftviskosität und einer Xylemviskoelastizität umfasst; und
- Bestimmen einer oder mehrerer Spitzen in wenigstens einem Teil des emissionsbezogenen Signals im Frequenzbereich, wobei der physikalische Gefäßparameter basierend auf den ein oder mehreren Spitzen bestimmt wird, und wobei der physikalische Gefäßparameter eines oder mehrere von einer Xylemgefäß-Elementlänge und einem Elastizitätsmodul umfasst.

7. Verfahren gemäß einem der vorstehenden Ansprüche, wobei das Verfahren ferner eines oder mehrere der folgenden umfasst:

- Steuern einer Pflanzenkultivierungsbedingung basierend auf dem physikalischen Gefäßparameter, wobei die Pflanzenkultivierungsbedingung aus der Gruppe ausgewählt ist, die ein Bewässerungsregime, ein Beleuchtungsregime und ein Nährstoffregime umfasst; und
- Bestimmen des physikalischen Gefäßparameters für mehrere Gefäßpflanzen (10), wobei das Verfahren umfasst, eine oder mehrere Gefäßpflanzen (10) der mehreren Gefäßpflanzen (10) für die Zucht auszuwählen, basierend auf den physikalischen Gefäßparametern.

8. Verfahren gemäß einem der vorstehenden Ansprüche, wobei eines oder mehrere der folgenden gelten:

- die akustische Emissionsausstrahlung (121) wird auf natürliche Weise durch die Gefäßpflanze (10) ausgestrahlt;
- die Gefäßpflanze (10) befindet sich in einem Stresszustand;
- die Gefäßpflanze (10) befindet sich in einem Stresszustand infolge von Trockenheit; und
- das Gefäßgewebe (20) ist ein Xylemgewebe in einem Pflanzenstängel (11) oder in einem Pflanzenzweig (12).

9. System (1000) zum Bestimmen eines physikalischen Gefäßparameters von Gefäßgewebe (20) in einer Gefäßpflanze (10), wobei das System (100) eine akustische Strahlungsvorrichtung (100) und ein Steuerungssystem (300) umfasst, wobei das System (1000) einen Betriebsmodus aufweist, wobei der Betriebsmodus umfasst:

- eine Detektionsstufe, die umfasst, dass die akustische Strahlungsvorrichtung (100) akustische Emissionsausstrahlung (121) von der Gefäßpflanze (10) erfasst und ein emissionsbezogenes Signal an das Steuerungssystem (300) bereitstellt; und
- eine Analysestufe, die umfasst, dass das Steuerungssystem (300) den physikalischen Gefäßparameter basierend auf dem emissionsbezogenen Signal bestimmt, wobei der physikalische Gefäßparameter eine Elastizität oder eine Gefäßdimension umfasst, und dass das Steuerungssystem (300) wenigstens einen Teil des emissionsbezogenen Signals an ein Modell von Biegemoden eines zylindrischen Strahls anpasst und den physikalischen Gefäßparameter basierend auf dem Modell bestimmt.

10. System (1000) gemäß Anspruch 9, wobei der Betriebsmodus ferner eines oder mehrere der folgenden umfasst:

- eine Erregungsstufe, die umfasst, dass die akustische Strahlungsvorrichtung (100) die Gefäßpflanze (10) mit akustischer Erregungsstrahlung (111) beaufschlagt, wobei die akustische Erregungsstrahlung eine Strahlung mit einer Frequenz umfasst, die aus dem Bereich von 1-250 kHz ausgewählt ist, wobei optional die akustische Erregungsstrahlung breitbandige Strahlung mit einer oder mehreren Frequenzen im Bereich von 10-150 kHz umfasst; und
- eine Ausführungsstufe, wobei die Ausführungsstufe umfasst, dass das Steuerungssystem eine Pflanzenkultivierungsbedingung basierend auf dem physikalischen Gefäßparameter steuert, wobei die Pflanzenkultivierungsbedingung aus der Gruppe ausgewählt ist, die ein Bewässerungsregime, ein Beleuchtungsregime und ein Nährstoffregime umfasst.

11. System (1000) gemäß Anspruch 10, wobei das System (1000) eine Stängelhalterung (1100) umfasst, die dafür ausgelegt ist, die akustische Strahlungsvorrichtung (100) an einem Pflanzenstängel (11) der Gefäßpflanze (10) zu befestigen, wobei der Betriebsmodus umfasst, den Pflanzenstängel (11) mit akustischer Erregungsstrahlung (111) zu beaufschlagen und akustische Emissionsausstrahlung (121) vom Pflanzenstängel (11) zu erfassen.

12. System (1000) gemäß einem der vorstehenden Ansprüche 10-11, wobei die Erregungsstufe umfasst, dass die akustische Strahlungsvorrichtung (100) die akustische Erregungsstrahlung (111) über einen Impuls bereitstellt, wobei eines oder mehrere der folgenden gelten:

- der Impuls hat eine Impulsdauer, die größer ist als eine charakteristische Einschwingzeit des Gefäßgewebes (20) ; und
- der Impuls hat eine Impulsdauer, die kleiner ist als die Laufzeit der akustischen Erregungsstrahlung durch das Gefäßgewebe (20).

13. System (1000) gemäß einem der vorstehenden Ansprüche 10-12, wobei:

- die Erregungsstufe umfasst, dass die akustische Strahlungsvorrichtung (100) einen Erregungsfrequenzhub von einer ersten Frequenz zu einer zweiten Frequenz bereitstellt; und
- die Detektionsstufe umfasst, dass die akustische Strahlungsvorrichtung (100) einen Erregungsfrequenzhub von der ersten Frequenz zur zweiten Frequenz bereitstellt;

wobei die erste Frequenz und die zweite Frequenz aus dem Bereich von 1-250 kHz ausgewählt sind.

14. System (1000) gemäß Anspruch 9-13, wobei das Gefäßgewebe (20) eine Längsachse (A) aufweist und wobei das System (1000) ferner eine Messstelle (1010) umfasst, die dafür ausgelegt ist, die Gefäßpflanze (10) aufzunehmen, wobei:

- die akustische Strahlungsvorrichtung (100) eine axiale Strahlungsdetektionsvorrichtung (120a) umfasst, die an einer ersten Stelle angeordnet ist, die axial entlang der Längsachse (A) angeordnet ist; und/oder
- die akustische Strahlungsvorrichtung (100) eine radiale Strahlungsdetektionsvorrichtung (120b) umfasst, die an einer zweiten, senkrecht zur Längsachse (A) angeordneten Stelle angeordnet ist.

15. System (1000) gemäß einem der vorstehenden Ansprüche 9-14, wobei die Analysestufe eines oder mehrere der folgenden umfasst:

- das Steuerungssystem (100) passt eine exponentielle Abklingkurve an wenigstens einen Teil des emissionsbezogenen Signals an und bestimmt einen Abklingparameter, wobei der physikalische Gefäßparameter basierend auf dem Abklingparameter bestimmt wird, wobei der physikalische Gefäßparameter eines oder mehrere von einem Xylemgefäßradius, einer Saftviskosität und einer Xylemviskoelastizität umfasst; und
- das Steuerungssystem (300) bestimmt eine oder mehrere Spitzen in wenigstens einem Teil des emissionsbezogenen Signals im Frequenzbereich, wobei der physikalische Gefäßparameter basierend auf den ein oder mehreren Spitzen bestimmt wird und wobei der physikalische Gefäßparameter eines oder mehrere von einer Xylemgefäß-Elementlänge und einem Elastizitätsmodul umfasst.

## Revendications

1. Procédé de détermination d'un paramètre physique de vaisseau d'un tissu vasculaire (20) dans une plante vasculaire (10), le procédé comprenant :

   - une étape de détection comprenant la détection d'un rayonnement d'émission acoustique (121) provenant de la plante vasculaire (10) et la fourniture d'un signal lié à l'émission ; et
   - une étape d'analyse comprenant la détermination du paramètre physique de vaisseau sur la base du signal lié à l'émission, le paramètre physique de vaisseau comprenant une élasticité ou une dimension de vaisseau, et l'étape d'analyse comprenant l'ajustement d'au moins une partie du signal lié à l'émission à un modèle de modes de flexion d'un faisceau cylindrique, et la détermination du paramètre physique de vaisseau sur la base du modèle.

2. Procédé selon l'une quelconque des revendications précédentes, le procédé comprenant :

   - une étape d'excitation comprenant la fourniture d'un rayonnement d'excitation acoustique (111) à la plante vasculaire (10), le rayonnement d'excitation acoustique (111) comprenant un rayonnement ayant une fréquence choisie dans la plage de 1 à 250 kHz, et éventuellement le rayonnement d'excitation acoustique (111) comprenant un rayonnement à large bande ayant une ou plusieurs fréquences dans la plage de 10 à 150 kHz.

3. Procédé selon la revendication 2, l'étape d'excitation comprenant la fourniture du rayonnement d'excitation acoustique (111) par l'intermédiaire d'une impulsion, une ou plusieurs des situations s'appliquant parmi :

   - l'impulsion a une durée d'impulsion supérieure à un temps de stabilisation caractéristique dû à l'amortissement du tissu vasculaire (20) ; et
   - l'impulsion a une durée inférieure au temps de vol du rayonnement d'excitation acoustique à travers le tissu vasculaire (20).

4. Procédé selon l'une quelconque des revendications précédentes 2 et 3, une ou plusieurs des situations s'appliquant parmi :

   - l'étape d'excitation comprend un balayage de fréquence d'excitation d'une première fréquence à une seconde fréquence ; et
   - l'étape de détection comprend un balayage de fréquence d'émission de la première fréquence à la seconde fréquence ;

   la première fréquence et la seconde fréquence étant choisies dans la plage de 1 à 250 kHz.

5. Procédé selon l'une quelconque des revendications précédentes, le tissu vasculaire (20) ayant un axe longitudinal (A), et l'étape de détection comprenant la détection de :

   - un rayonnement d'émission acoustique (121) provenant de la plante vasculaire (10) au niveau d'un premier emplacement agencé axialement par rapport à l'axe longitudinal (A), et/ou
   - un rayonnement d'émission acoustique (121) provenant de la plante vasculaire (10) au niveau d'un second emplacement agencé perpendiculairement à l'axe longitudinal (A).

**6.** Procédé selon l'une quelconque des revendications précédentes, l'étape d'analyse comprenant une ou plusieurs des opérations suivantes :

- l'ajustement d'une courbe de décroissance exponentielle à au moins une partie du signal lié à l'émission et la détermination d'un paramètre de décroissance, le paramètre physique de vaisseau étant déterminé sur la base du paramètre de décroissance, le paramètre physique de vaisseau comprenant un ou plusieurs éléments parmi un rayon de vaisseau de xylème, une viscosité de sève et une viscoélasticité de xylème ; et
- la détermination d'un ou plusieurs pics dans au moins une partie du signal lié à l'émission dans le domaine fréquentiel, le paramètre physique de vaisseau étant déterminé sur la base du ou des pics, et le paramètre physique de vaisseau comprenant un ou plusieurs éléments parmi une longueur d'élément de vaisseau de xylème et un module de Young.

**7.** Procédé selon l'une quelconque des revendications précédentes, le procédé comprenant en outre une ou plusieurs des opérations suivantes :

- la commande d'une condition de culture de plante sur la base du paramètre physique de vaisseau, la condition de culture de plante étant choisie dans le groupe comprenant un régime d'arrosage, un régime d'éclairage et un régime de nutriments ; et
- la détermination du paramètre physique de vaisseau pour une pluralité de plantes vasculaires (10), le procédé comprenant la sélection d'une ou plusieurs plantes vasculaires (10) parmi la pluralité de plantes vasculaires (10) pour la reproduction sur la base des paramètres physique de vaisseau.

**8.** Procédé selon l'une quelconque des revendications précédentes, une ou plusieurs des situations s'appliquant parmi :

- le rayonnement d'émission acoustique (121) est naturellement émis par la plante vasculaire (10) ;
- la plante vasculaire (10) est dans un état de stress ;
- la plante vasculaire (10) est dans un état de stress à la suite d'une sécheresse ; et
- le tissu vasculaire (20) est un tissu de xylème dans une tige végétale (11) ou dans une branche végétale (12).

**9.** Système (1000) pour déterminer un paramètre physique de vaisseau d'un tissu vasculaire (20) dans une plante vasculaire (10), le système (100) comprenant un dispositif de rayonnement acoustique (100) et un système de commande (300), le système (1000) ayant un mode de fonctionnement, le mode de fonctionnement comprenant :

- une étape de détection comprenant la détection par le dispositif de rayonnement acoustique (100) du rayonnement d'émission acoustique (121) provenant de la plante vasculaire (10) et fournissant un signal lié à l'émission au système de commande (300), et
- une étape d'analyse comprenant la détermination par le système de commande (300) du paramètre physique de vaisseau sur la base du signal lié à l'émission, le paramètre physique de vaisseau comprenant une élasticité ou une dimension de vaisseau, et le système de commande (300) ajustant au moins une partie du signal lié à l'émission à un modèle de modes de flexion d'un faisceau cylindrique, et déterminant le paramètre physique de vaisseau sur la base du modèle.

**10.** Système (1000) selon la revendication 9, le mode de fonctionnement comprenant en outre un ou plusieurs des éléments suivants :

- une étape d'excitation comprenant la fourniture par le dispositif de rayonnement acoustique (100) d'un rayonnement d'excitation acoustique (111) à la plante vasculaire (10), le rayonnement d'excitation acoustique comprenant un rayonnement ayant une fréquence choisie dans la plage de 1 à 250 kHz, éventuellement le rayonnement d'excitation acoustique comprenant un rayonnement à large bande ayant une ou plusieurs fréquences dans la plage de 10 à 150 kHz ; et
- une étape d'exécution, l'étape d'exécution comprenant la commande par le système de commande d'une condition de culture de plante sur la base du paramètre physique de vaisseau, la condition de culture de plante étant choisie dans le groupe comprenant un régime d'arrosage, un régime d'éclairage et un régime de nutriments.

**11.** Système (1000) selon la revendication 10, le système (1000) comprenant un support de tige (1100) configuré pour fixer le dispositif de rayonnement acoustique (100) à une tige végétale (11) de la plante vasculaire (10), le mode de fonctionnement comprenant la fourniture d'un rayonnement d'excitation acoustique (111) à la tige végétale (11) et la détection d'un rayonnement d'émission acoustique (121) à partir de la tige végétale (11).

**12.** Système (1000) selon l'une quelconque des revendications précédentes 10 et 11, l'étape d'excitation comprenant la fourniture par le dispositif de rayonnement acoustique (100) du rayonnement d'excitation acoustique (111) par l'intermédiaire d'une impulsion, une ou plusieurs des situations s'appliquant parmi :

- l'impulsion a une durée d'impulsion supérieure à un temps de stabilisation caractéristique du tissu vasculaire (20) ; et
- l'impulsion a une durée inférieure au temps de vol du rayonnement d'excitation acoustique à travers le tissu vasculaire (20).

**13.** Système (1000) selon l'une quelconque des revendications précédentes 10 à 12 :

- l'étape d'excitation comprenant la fourniture par le dispositif de rayonnement acoustique (100) d'un balayage de fréquence d'excitation d'une première fréquence à une seconde fréquence ; et
- l'étape de détection comprenant la fourniture par le dispositif de rayonnement acoustique (100) d'un balayage de fréquence d'émission de la première fréquence à la seconde fréquence ; la première fréquence et la seconde fréquence étant choisies dans la plage de 1 à 250 kHz.

**14.** Système (1000) selon la revendication 9 à 13, le tissu vasculaire (20) ayant un axe longitudinal (A), et le système (1000) comprenant en outre un site de mesure (1010) configuré pour accueillir la plante vasculaire (10) :

- le dispositif de rayonnement acoustique (100) comprenant un dispositif de détection de rayonnement axial (120a) agencé au niveau d'un premier emplacement agencé axialement le long de l'axe longitudinal (A) ; et/ou
- le dispositif de rayonnement acoustique (100) comprenant un dispositif de détection de rayonnement radial (120b) agencé au niveau d'un second emplacement perpendiculairement à l'axe longitudinal (A).

**15.** Système (1000) selon l'une quelconque des revendications précédentes 9 à 14, l'étape d'analyse comprenant une ou plusieurs opérations parmi :

- le système de commande (300) ajustant une courbe de décroissance exponentielle à au moins une partie du signal lié à l'émission et déterminant un paramètre de décroissance, le paramètre physique de vaisseau étant déterminé sur la base du paramètre de décroissance, le paramètre physique de vaisseau comprenant un ou plusieurs éléments parmi un rayon de vaisseau de xylème, une viscosité de sève et une viscoélasticité de xylème ; et
- le système de commande (300) déterminant un ou plusieurs pics dans au moins une partie du signal lié à l'émission dans le domaine fréquentiel, le paramètre physique de vaisseau étant déterminé sur la base du ou des pics, et le paramètre physique de vaisseau comprenant un ou plusieurs éléments parmi une longueur d'élément de vaisseau de xylème et un module de Young.

FIG. 1

FIG. 2

FIG. 3A

FIG. 3B

FIG. 3C

FIG. 3D

FIG. 3E

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 2359678 A1
- EP 3667312 A1

### Non-patent literature cited in the description

- **PAN et al.** A comparison of two methods for measuring vessel length in woody plants. *Plant, Cell and Environment,* 2015
- **GUILLAUME et al.** Changes in ultrasound velocity and attenuation indicate freezing of xylem sap. *Agricultural and Forest Meteorology,* 2014
- **BRODERSEN et al.** In vivo visualization of the final stages of xylem vessel refilling in grapevine (Vitis vinifera) stems. *New Phytologist,* 2017
- **CHATELET et al.** Xylem Structure and Connectivity in Grapevine (Vitis vinifera) Shoots Provides a Passive Mechanism for the Spread of Bacteria in Grape Plants. *Annals of Botany,* 2006